(19) Europäisches Patentamt
European Patent Office
Office européen des brevets

(11) Veröffentlichungsnummer: **0 344 572 B1**

(12) **EUROPÄISCHE PATENTSCHRIFT**

(45) Veröffentlichungstag der Patentschrift: **08.12.93**

(51) Int. Cl.⁵: **A61K 37/64**, A61K 45/06, A61K 37/02

(21) Anmeldenummer: **89109209.0**

(22) Anmeldetag: **23.05.89**

Die Akte enthält technische Angaben, die nach dem Eingang der Anmeldung eingereicht wurden und die nicht in dieser Patentschrift enthalten sind.

# (54) Kombination von Angiotensin-Converting-Enzyme-Hemmern mit Kaliumkanal-Modulatoren sowie deren Verwendung in Arzneimitteln.

(30) Priorität: **28.05.88 DE 3818245**

(43) Veröffentlichungstag der Anmeldung:
**06.12.89 Patentblatt 89/49**

(45) Bekanntmachung des Hinweises auf die Patenterteilung:
**08.12.93 Patentblatt 93/49**

(84) Benannte Vertragsstaaten:
**AT BE CH DE ES FR GB GR IT LI LU NL SE**

(56) Entgegenhaltungen:
**EP-A- 0 076 075** **EP-A- 0 107 423**
**EP-A- 0 120 427** **EP-A- 0 120 428**
**EP-A- 0 158 157** **EP-A- 0 180 785**
**EP-A- 0 219 782** **EP-A- 0 256 402**
**EP-A- 0 257 485** **EP-A- 0 265 685**
**DD-A- 239 525** **FR-A- 2 605 885**

(73) Patentinhaber: **HOECHST AKTIENGESELLSCHAFT**
**D-65926 Frankfurt(DE)**

(72) Erfinder: **Becker, Reinhard, Dr.**
**Humboldtstrasse 17**
**D-6000 Wiesbaden(DE)**
Erfinder: **Henning, Rainer, Dr.**
**Rotenhofstrasse 31**
**D-6234 Hattersheim am Main(DE)**
Erfinder: **Urbach, Hansjörg, Dr.**
**Le Lavandoustrasse 41**
**D-6242 Kronberg/Taunus(DE)**
Erfinder: **Teetz, Volker, Dr.**
**An der Tann 20**
**D-6238 Hofheim am Taunus(DE)**
Erfinder: **Englert, Heinrich Christian, Dr.**
**Stormstrasse 13**
**D-6238 Hofheim am Taunus(DE)**
Erfinder: **Mania, Dieter, Dr.**
**Berliner Ring 5**
**D-6233 Kelkheim (Taunus)(DE)**

**EP 0 344 572 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft eine Kombination von Angiotensin-Converting-Enzyme-Hemmern (ACE-Hemmer) mit Kaliumkanal-Modulatoren sowie ihre Verwendung in Arzneimitteln, insbesondere in blutdrucksenkenden Arzneimitteln.

ACE-Hemmer sind Verbindungen, die die Umwandlung von Angiotensin I in das pressorisch wirksame Angiotensin II verhindern. Solche Verbindungen sind beispielsweise in den folgenden Patentanmeldungen oder Patenten beschrieben:
US-PS 4 350 633, US-PS 4 344 949, US-PS 4 294 832, US-PS 4 350 704, EP-A 50 800, EP-A 31 741, EP-A 51 020, EP-A 49 658, EP-A 49 605, EP-A 29 488, EP-A 46 953, EP-A 52 870, EP-A 72 022, EP-A 84 164, EP-A 89 637, EP-A 90 341, EP-A 90 362, EP-A 105 102, EP-A 109 020, EP-A 111 873, EP-A 113 880.
Ihre blutdrucksenkende Wirkung ist gut dokumentiert. Kaliumkanal-Modulatoren sind solche Verbindungen, die den Ausstrom von Kaliumionen aus Zellen, insbesondere glatten Muskelzellen beeinflussen und dadurch zu einer Membranhyperpolarisation führen. Solche Verbindungen sowie ihre blutdrucksenkende Wirkung sind in Patentanmeldungen und Publikationen niedergelegt, beispielsweise in J. Med. Chem. 29 (1986) 2194-2201 sowie den europäischen Patentanmeldungen EP-A 76 075, EP-A 107 423, EP-A 120 427 und EP-A 120 428.

Aus EP-A 180 785 sind Kombinationen von monocyclischen ACE-Inhibitoren mit speziellen Dihydropyridinderivaten bekannt, in der prioritätsälteren, nicht-veröffentlichten EP-A 265 685 wird eine Kombination von ACE-Inhibitoren mit Calciumantagonisten vorgestellt.

Beide Substanzgruppen der erfindungsgemaßen Kombination greifen in verschiedene Blutdruckregulationssysteme ein. Dabei wird überraschenderweise bei einer kombinierten Anwendung der Effekt des einen Kombinationspartners durch den jeweiligen anderen Partner gesteigert. Dies führt bei einer kombinierten Anwendung zu einer Verringerung der Dosis der jeweiligen Kombinationspartner, verglichen mit der Einzelanwendung. Dadurch kann das Auftreten der für die beiden Substanzklassen bekannten Nebenwirkungen verringert bzw. vermieden werden. Kombinationen von Vertretern dieser Wirkstoffklassen sind bisher nicht beschrieben worden.

Als ACE-Hemmer kommen die folgenden Verbindungen der Formel I oder deren physiologisch verträgliche Salze in Betracht:

$$R^3OOC - \overset{*}{\underset{R^4}{CH}} - \underset{R^5}{N} - \underset{O}{\overset{\|}{C}} - \overset{*}{\underset{R^1}{CH}} - NH - \overset{*}{\underset{COOR^2}{CH}} - (CH_2)_n - R \qquad (I)$$

in welcher

n = 1 oder 2 ist,

R = Wasserstoff,
einen gegebenenfalls substituierten aliphatischen Rest mit 1-8 C-Atomen,
einen gegebenenfalls substituierten alicyclischen Rest mit 3-9 C-Atomen,
einen gegebenenfalls substituierten aromatischen Rest mit 6-12 C-Atomen,
einen gegebenenfalls substituierten araliphatischen Rest mit 7-14 C-Atomen,
einen gegebenenfalls substituierten alicyclischaliphatischen Rest mit 7-14 C-Atomen,
einen Rest $OR^a$ oder $SR^a$, worin

$R^a$ für einen gegebenenfalls substituierten aliphatischen Rest mit 1-4 C-Atomen, für einen gegebenenfalls substituierten aromatischen Rest mit 6-12 C-Atomen oder einen gegebenenfalls substituierten heteroaromatischen Rest mit 5-12 Ringatomen steht,

$R^1$ Wasserstoff, einen gegebenenfalls substituierten aliphatischen Rest mit 1-6 C-Atomen,
einen gegebenenfalls substituierten alicyclischen Rest mit 3-9 C-Atomen,
einen gegebenenfalls substituierten alicyclischaliphatischen Rest mit 4-13 C-Atomen,
einen gegebenenfalls substituierten aromatischen Rest mit 6-12 C-Atomen,
einen gegebenenfalls substituierten araliphatischen Rest mit 7-16 C-Atomen,
einen gegebenenfalls substituierten heteroaromatischen Rest mit 5-12 Ringatomen oder
die erforderlichenfalls geschützte Seitenkette einer natürlich vorkommenden α-Aminosäure bedeuten,

$R^2$ und $R^3$ gleich oder verschieden sind und Wasserstoff,

einen gegebenenfalls substituierten aliphatischen Rest mit 1-6 C-Atomen,
einen gegebenenfalls substituierten alicyclischen Rest mit 3-9 C-Atomen,
einen gegebenenfalls substituierten aromatischen Rest mit 6-12 C-Atomen,
einen gegebenenfalls substituierten araliphatischen Rest mit 7-16 C-Atomen bedeuten und

$R^4$ und $R^5$ zusammen mit den sie tragenden Atomen ein heterocyclisches bi-cyclisches Ringsystem aus der folgenden Gruppe:
Tetrahydroisochinolin (A); Decahydroisochinolin (B); Octahydroindol (C); Octahydrocyclopenta[b]pyrrol (D)
die alle gegebenenfalls substituiert sein können. Bevorzugt sind jedoch die unsubstituierten Systeme.

Bei den Verbindungen, die mehrere chirale Atome besitzen, kommen alle möglichen Diastereomere als Racemate oder Enantiomere, oder Gemische verschiedener Diastereomare in Betracht.

Die in Betracht kommenden cyclischen Aminosäureester weisen die folgenden Strukturformeln auf.

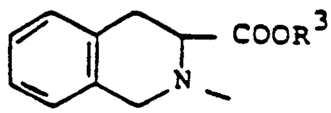

A

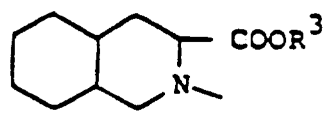

B

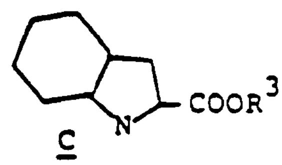

C

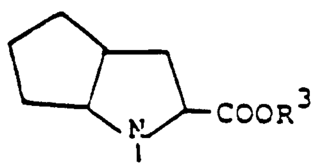

D

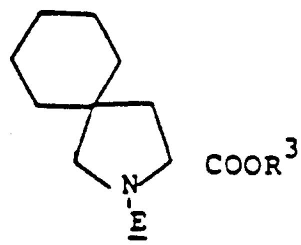

E

Besonders bevorzugt sind ACE-Hemmer der Formel I, in der

n = 1 oder 2 ist

R Wasserstoff,
Alkyl mit 1-8 C-Atomen,
Alkenyl mit 2-6 C-Atomen,
Cycloalkyl mit 3-9 C-Atomen,
Aryl mit 6-12 C-Atomen,
das durch $(C_1-C_4)$-Alkyl, $(C_1-C_4)$-Alkoxy, Hydroxy, Halogen, Nitro, Amino, Aminomethyl, $(C_1-C_4)$-Alkylamino,Di-$(C_1-C_4)$-alkylamino, $(C_1-C_4)$-Alkanoylamino, Methylendioxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-$(C_1-C_4)$-alkyl,
$(C_1-C_4)$-Alkanoylamino-$(C_1-C_4)$-alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,

$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4)$-alkylthio,
das im Arylteil wie oben bei Aryl beschrieben substituiert sein kann,
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_6-C_{12})$-Aryloxy-$(C_1-C_4)$-alkyl,
das im Arylteil wie oben bei Aryl beschrieben substituiert sein kann oder
$(C_6-C_{12})$-Aryl-$(C_1-C_4)$-alkoxy,
das im Arylteil wie oben bei Aryl beschrieben substituiert sein kann, bedeutet,

$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6-12 C-Atomen, das wie oben bei R beschrieben substituiert sein kann, $(C_6-C_{12})$-Aryl-$(C_1-C_4)$-alkyl oder $(C_7-C_{13})$-Aroyl-($C_1$ oder $C_2$)alkyl
die beide wie das vorstehende Aryl substituiert sein können,
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer nattürlich vorkommenden α-Aminosäure $R^1$-$CH(NH_2)$-$COOH$ bedeutet,

$R^2$ und $R^3$ gleich oder verschieden sind und Wasserstoff,
Alkyl mit 1-6 C-Atomen,
Alkenyl mit 2-6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy-$(C_1-C_4)$-alkyl,
Aryl mit 6-12 C-Atomen,
$(C_6-C_{12})$-Aryl-$(C_1-C_4)$-alkyl,
$(C_3-C_9)$-Cycloalkyl oder
$(C_3-C_9)$-Cycloalkyl-$(C_1-C_4)$-alkyl
bedeuten und

$R^4$ und $R^5$ die oben angegebene Bedeutung haben.

Besonders bevorzugt sind Verbindungen der Formel I, in der

n = 1 oder 2 ist,

R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino$(C_1-C_4)$-alkyl, $(C_1-C_4)$-Alkoxy-carbonylamino-$(C_1-C_4)$-alkyl, $(C_6-C_{12})$-Aryl-$(C_1-C_4)$-alkoxycarbonylamino$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,

$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloal-

kenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$-$C_4$)-alkyl oder ($C_7$-$C_{13}$)-Aroyl($C_1$-$C_2$)-alkyl, die beide wie vorstehend definiert im Arylrest substituiert sein können, ein mono- bzw. bicyclischer Heterocyclen Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, ($C_1$-$C_4$)-Alkyl oder Benzyl bedeuten und

$R^4$ und $R^5$ die oben angegebene Bedeutung haben.

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy oder Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen in der Peptidchemie übliche Gruppen bevorzugt (vgl. Houben-Weyl, Bd. XV/1 und XV/2). Im Falle, daß $R^1$ die geschützte LysinSeitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen bevorzugt ($C_1$-$C_6$)-Alkyl, insbesondere Methyl oder Ethyl in Frage.

Insbesondere bevorzugt sind die Verbindungen 2-[N-(1-S-Ethoxycarbonyl-3-phenylpropyl)-S-alanyl]-(1S,3S,5S)-2-azabicyclo[3.3.0]octan-3-carbonsäure (Ramipril), 1-[N-(1-S-Ethoxycarbonyl-3-phenylpropyl)-S-alanyl]-(2S,3aR,7aS)-octahydro[1H]indol-2-carbonsäure (Trandolapril) sowie 2-[N-(1-S-Ethoxycarbonyl-3-phenylpropyl)-S-alanyl]-1.2.3.4-tetrahydroisochinolin-3-S-carbonsäure (Quinapril).

Die Herstellung der ACE-Hemmer der Formel I ist in den auf Seite 1 genannten Patentanmeldungen oder Patentschriften beschrieben.

Als Kaliumkanal-Modulatoren kommen Verbindungen der Formel II in Betracht,

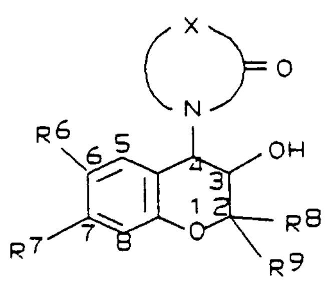

(II)

in welcher

$R^6$ für CN, $NO_2$, $SO_n$-($C_1$-$C_6$)-Alkyl, $SO_n$-Ar, steht; wobei n = 1 oder 2, Ar für ein aromatisches oder heteroaromatisches System steht, das unsubstituiert oder durch 1 bis 3 gleiche oder verschiedene Reste ($C_1$-$C_2$)-Alkyl, ($C_1$-$C_2$)-Alkoxy, Halogen, Trifluormethyl, CN, $NO_2$, CO-($C_1$-$C_2$)-Alkyl, $SO_p$-($C_1$-$C_2$) Alkyl, substituiert ist und p für 1 oder 2 steht,

$R^7$ für Wasserstoff, Hydroxy, ($C_1$-$C_2$)-Alkoxy, ($C_1$-$C_2$)-Alkyl, Halogen oder $NR^{10}R^{11}$ steht, wobei $R^{10}$ und $R^{11}$ gleich oder verschieden sind und für Wasserstoff, ($C_1$-$C_2$)-Alkyl oder ($C_1$-$C_2$)-Alkylcarbonyl stehen, wobei die oben genannten Bedeutungen von $R^6$ und $R^7$

auch vertauscht sein können,

$R^8$ und $R^9$ gleich oder verschieden sind und für Alkyl mit 1-4 C-Atomen stehen,

X für eine Kette $(CH_2)m$ steht, die unsubstituiert ist oder durch mindestens 1 und höchstens 2m-1 $(C_1-C_2)$-Alkylgruppen substituiert ist, sowie durch ein Heteroatom Y in der Bedeutung von O, $NR^{12}$, S unterbrochen sein kann und $R^{12}$ H oder $(C_1-C_4)$-Alkyl bedeutet und m für 2, 3 oder 4 steht, wobei die Konfiguration an $C_3$ und $C_4$ stets entgegengesetzt ist.

Unter einem aromatischen System Ar wird vorzugsweise Phenyl, Naphthyl oder Biphenylyl verstanden, ein 5 oder 6-gliedriges heteroaromatisches System Ar ist vorzugweise ein Rest eines 5 oder 6-gliedrigen O-, N- und/oder S-heterocyclischen Ringes, insbesondere Furyl, Thienyl, Isothiazolyl, Oxazolyl, Isoxazolyl, Pyrazolyl, Imidazolyl, Thiazolyl, Pyridyl, Pyrazinyl, Pyrimidinyl, Pyridazinyl, Thiazinyl.

Unter Halogen wird F, Cl, Br oder J, vorzugsweise F und Cl verstanden.

Die C-Atome 3 und 4 des 3,4-Dihydro-2H-benzo[b]pyransystems (nachfolgend auch als "Chromansystem" bezeichnet) der Formel II sind asymmetrisch substituiert. Die Erfindung betrifft nur solche Verbindungen, die an diesen Zentren entgegengesetzte Konfigurationen aufweisen. Dies bedeutet, daß der Lactamring als Substituent an C-4 und die OH-Gruppe an C-3 stets "trans" zueinander orientiert sind. Die oben genannte Definition von X bedeutet, daß außerdem der Lactamring mindestens ein, höchstens jedoch m (mit m in der eingangs erwähnten Definition) chirale C-Atome enthält. Die Erfindung betrifft dabei sowohl Verbindungen mit R-als auch S-konfigurierten Zentren. Gleiches gilt für den Fall, daß $R^6$, $R^7$, $R^8$ und $R^9$ Asymmetriezentren enthalten oder selbst als Substituenten ein Asymmetriezentrum erzeugen. Die Verbindungen können dann als optische Isomere, als Diastereoisomere, als Racemate oder als Gemisch derselben vorliegen.

Bevorzugt sind Verbindungen der Formel II, in denen

$R^6$, $R^7$, $R^8$, $R^9$ die oben genannten Bedeutungen aufweisen und X für eine Kette $(CH_2)_m$ steht; die unsubstituiert ist oder durch eine $(C_1-C_2)$-Alkylgruppe substituiert ist, sowie durch ein Heteroatom Y, das für O, S oder $NR^{12}$ steht mit $R^{12}$ in der Bedeutung von Wasserstoff oder $(C_2-C_4)$-Alkyl, unterbrochen sein kann und wobei m für 2, 3 oder 4 steht.

Weiter bevorzugt sind Verbindungen der Formel II, in welcher

$R^6$ bis $R^9$ die oben genannten Bedeutungen aufweisen und X für eine Kette $(CH_2)_m$ steht, die unsubstituiert ist oder durch eine $(C_1-C_2)$-Alkylgruppe substituiert ist wobei m für 3 oder 4 steht.

Ganz besonders bevorzugt sind dabei solche Verbindungen der Formel II, bei denen $R^6$ bis $R^9$ die oben genannten Bedeutungen aufweisen und X für eine Kette $(CH_2)_m$ steht, wobei m für 3 oder 4 steht, die durch eine $(C_1-C_2)$-Alkylgruppe an dem C-Atom substituiert ist, das dem N-Atom des Lactamringes benachbart ist.

Insbesondere bevorzugt sind Verbindungen, bei denen $R^6$ bis $R^9$ die oben genannten Bedeutungen aufweisen und X für eine Kette $(CH_2)_m$ steht mit m = 3 oder 4, die unsubstituiert ist oder durch eine $(C_1-C_2)$-Alkylgruppe an dem C-Atom substituiert ist, das dem N-Atom des Lactamringes benachbart ist, und zwar derart, daß die Konfiguration dieses C-Atoms dieselbe ist wie das des C-4 Atoms des Chromansystems.

Ebenfalls ganz besonders bevorzugt sind Verbindungen der Formel II bei denen

$R^6$ für CN oder $SO_2-CH_3$ und $R^7$ für Wasserstoff steht, $R^8$ und $R^9$ gleich oder verschieden sind und für Alkyl mit 1 bis 2 C-Atomen stehen,

X für eine Kette $(CH_2)_m$ steht mit m = 3 oder 4, die unsubstituiert ist oder durch eine $(C_1-C_2)$-Alkylgruppe an dem C-Atom substituiert ist, das dem N-Atom des Lactamringes benachbart ist, und zwar derart, daß die Konfiguration dieses C-Atoms dieselbe ist wie das des C-4 Atoms im Chromansystem;

ebenso bevorzugt sind Verbindungen II, bei denen $R^6$ für $SO_2$-Ar steht mit Ar in der Bedeutung von Phenyl, das unsubstituiert oder durch 1 bis 3 Substituenten wie oben erwähnt substituiert ist,

$R^7$ für Wasserstoff oder $OCH_3$ steht,

$R^8$ und $R^9$ gleich sind oder verschieden und für $(C_1-C_2)$-Alkyl stehen,

X für eine Kette $(CH_2)_m$ steht mit m = 3 oder 4, die unsubstituiert ist oder durch eine $(C_1-C_2)$-Alkylgruppe an dem C-Atom substituiert ist, das dem N-Atom des Lactamringes benachbart ist, und zwar derart, daß die Konfiguration dieses C-Atoms dieselbe ist wie die des C-4-Atoms im Chromansystems.

Bevorzugt sind auch Verbindungen, bei denen $R^6$ bis $R^9$ die oben genannten Bedeutungen aufweisen und X für eine Kette $(CH_2)_m$ steht mit m = 3 oder 4, die unsubstituiert ist oder durch eine $(C_1-C_2)$-Alkylgruppe an dem C-Atom substituiert ist, das dem N-Atom des Lactamringes benachbart ist, und zwar derart, daß die Konfiguration dieses C-Atoms entgegengesetzt ist zu der des C-4 Atom des Chromansystems.

Ebenfalls ganz besonders bevorzugt sind Verbindungen II, bei denen

$R^6$ für CN oder $SO_2-CH_3$ und $R^7$ für Wasserstoff,

$R^8$ und $R^9$ gleich oder verschieden sind und für Alkyl mit 1 bis 2 C-Atomen stehen,
X für eine Kette $(CH_2)_m$ steht mit m = 3 oder 4, die unsubstituiert ist oder durch eine $(C_1\text{-}C_2)$-Alkylgruppe an dem C-Atom substituiert ist, das dem N-Atom des Lactamringes benachbart ist, und zwar derart, daß die Konfiguration dieses C-Atoms entgegengesetzt ist zu der des C-4 Atoms im Chromansystem;
ebenso bevorzugt sind Verbindungen II, bei denen $R^6$ für $SO_2$-Ar steht mit Ar in der Bedeutung von Phenyl, das unsubstituiert oder durch 1 bis 3 Substituenten wie oben erwähnt substituiert ist,
$R^7$ für Wasserstoff oder $OCH_3$ steht,
$R^8$ und $R^9$ gleich sind oder verschieden und für $(C_1\text{-}C_2)$-Alkyl stehen,
X für eine Kette $(CH_2)_m$ steht mit m = 3 oder 4, die unsubstituiert ist oder durch eine $(C_1\text{-}C_2)$-Alkylgruppe an dem C-Atom substituiert ist, das dem N-Atom des Lactamringes benachbart ist, und zwar derart, daß die Konfiguration dieses C-Atoms entgegengesetzt ist zu der des C-4-Atoms im Chromansystems.

Von ganz besonderer Bedeutung sind die folgenden Kombinationen

| | |
|---|---|
| Ramipril + | (±)-6-Cyano-3,4-dihydro-2,2-dimethyl-trans-4-(2-oxo-1-pyrrolidinyl)-2H-benzo[b]pyran-3-ol (Chromakalim), |
| Ramipril + | 6-Cyano-3-hydroxy-2,2-dimethyl-4-(5-methyl-2-oxo-1-pyrrolidinyl)-3,4-dihydro-2H-benzo[b]pyran, |
| Ramipril + | 2,2-Dimethyl-3-hydroxy-6-phenylsulfonyl-4-(2-oxo-1-pyrrolidinyl)-3,4-dihydro-2H-benzo[b]pyran, |
| Trandolapril + | Chromakalim, |
| Trandolapril + | 6-Cyano-3-hydroxy-2,2-dimethyl-4-(5-methyl-2-oxo-1-pyrrolidinyl)-3,4-dihydro-2H-benzo [b]pyran, |
| Trandolapril + | 2,2-Dimethyl-3-hyroxy-6-phenylsulfonyl-4-(2-oxo-1-pyrrolidinyl)-3,4-dihydro-2H-benzo-[b]pyran, |
| Quinapril + | Chromakalim, |
| Quinapril + | 6-Cyano-3-hydroxy-2,2-dimethyl-4-(5-methyl-2-oxo-1-pyrrolidinyl)-3,4-dihydro-2H-benzo[b]pyran, |
| Quinapril + | 2,2-Dimethyl-3-hydroxy-6-phenylsulfonyl-4-(2-oxo-1-pyrrolidinyl)-3,4-dihydro-2H-benzo[b]pyran, |
| Ramipril + | (3S,4R)-6-Cyano-3,4-dihydro-2,2-dimethyl-4-(2-oxo-1-pyrrolidinyl)-2H-benzo[b]pyran-3-ol, |
| Trandolapril + | (3S,4R)-6-Cyano-3,4-dihydro-2,2-dimethyl-4-(2-oxo-1-pyrrolidinyl)-2H-benzo[b]pyran-3-ol, |
| Quinapril + | (3S,4R)-6-Cyano-3,4-dihydro-2,2-dimethyl-4-(2-oxo-1-pyrrolidinyl)-2H-benzo[b]pyran-3-ol, |
| Ramipril + | (3S,4R)-6-Phenylsulfonyl-3,4-dihydro-2,2-dimethyl-4-(2-oxo-1-pyrrolidinyl)-2H-benzo-[b]-pyran-3-ol, |
| Trandolapril + | (3S,4R)-6-Phenylsulfonyl-3,4-dihydro-2,2-dimethyl-4-(2-oxo-1-pyrrolidinyl)-2H-benzo-[b]pyran-3-ol, |
| Quinapril + | (3S,4R)-6-Phenylsulfonyl-3,4-dihydro-2,2-dimethyl-4-(2-oxo-1-pyrrolidinyl)-2H-benzo-[b]-pyran-3-ol |

sowie jeweils die physiologisch verträglichen Salze der genannten Einzelkomponente, falls solche gebildet werden können.

Kaliumkanal-Modulatoren können nach dem in J.Med. Chem. 29 (1986) 2194-2201, EP-A 76 075, EP-A 107 423, EP-A 120 427 und EP-A 120 428 beschriebenen Verfahren hergestellt werden.

Die Erfindung betrifft auch ganz allgemein Erzeugnisse, die enthalten:

a) einen ACE-Hemmer der Formel I oder dessen physiologisch verträgliches Salz und

b) einen Kaliumkanal-Modulator der Formel II oder dessen physiologisch verträgliches Salz

als Kombinationspräparat zur gleichzeitigen getrennten oder zeitlich abgestuften Anwendung bei der Behandlung des Bluthochdrucks.

In Tabelle 1 ist der mittlere artielle Blutdruck, gemessen an der wachen spontan hypertonen Ratte analog dem in Arzneimittelforschung 34 (II), S. 1419 (1984) beschriebenen Verfahren, aufgeführt.

Tabelle 1:

Mittlerer arterieller Blutdruck bei wachen spontan hypertonen Ratten nach oraler Gabe von

Gruppe 1: Placebo
Gruppe 2: 0,1 mg/kg Chromakalim
Gruppe 3: 0,3 mg/kg Chromakalim
Gruppe 4: 1,0 mg/kg Chromakalim
Gruppe 5: 0,1 mg/kg Chromakalim + 1,0 mg/kg Ramipril
Gruppe 6: 0,3 mg/kg Chromakalim + 1,0 mg/kg Ramipril

| t | mittlerer arterieller Druck [mm Hg] ± SEM (n = 6) Gruppe | | | | | |
|---|---|---|---|---|---|---|
| [min] | 1 | 2 | 3 | 4 | 5 | 6 |
| 0 | 164± 8 | 136± 8 | 153± 8 | 162± 6 | 166± 6 | 154± 3 |
| 2 | 158± 4 | 167±10 | 139±16 | 152± 9 | 158± 6 | 155± 7 |
| 3 | 158± 4 | 162± 9 | 134±16 | 124±10 | 151± 5 | 143± 8 |

Tabelle 1 Fortsetzung:

| t [min] | mittlerer arterieller Druck [mm Hg] ± SEM (n = 6) Gruppe | | | | | |
|---|---|---|---|---|---|---|
| | 1 | 2 | 3 | 4 | 5 | 6 |
| 5 | 161± 8 | 165± 8 | 111±12 | 107± 7 | 137± 4 | 120±13 |
| 10 | 163± 8 | 151±11 | 89± 9 | 84± 5 | 116± 6 | 91± 4 |
| 15 | 164± 8 | 141±12 | 89± 8 | 84± 4 | 103± 6 | 86± 3 |
| 20 | 161± 8 | 139±13 | 84± 7 | 79± 4 | 96± 7 | 82± 3 |
| 25 | 163± 8 | 138±13 | 88± 7 | 78± 3 | 94± 9 | 79± 3 |
| 30 | 160± 8 | 142±15 | 86± 6 | 82± 3 | 94± 8 | 83± 3 |
| 35 | 161± 8 | 139±14 | 87± 5 | 82± 3 | 106± 5 | 86± 2 |
| 40 | 159± 7 | 139±14 | 90± 4 | 82± 4 | 106± 5 | 88± 2 |
| 45 | 159± 7 | 141±13 | 92± 4 | 82± 4 | 109± 5 | 87± 3 |
| 50 | 158± 9 | 142±11 | 92± 4 | 84± 3 | 113± 4 | 91± 4 |
| 55 | 158± 9 | 143±11 | 98± 5 | 85± 3 | 118± 3 | 93± 4 |
| 60 | 162± 8 | 143±11 | 99± 4 | 87± 4 | 119± 3 | 95± 5 |
| 75 | 156± 9 | 144±13 | 108± 7 | 87± 4 | 124± 4 | 98± 5 |
| 90 | 161± 6 | 142±11 | 111± 8 | 90± 4 | 123± 4 | 96± 3 |
| 105 | 157± 6 | 139±12 | 113± 7 | 92± 5 | 114± 6 | 92± 3 |
| 120 | 153± 6 | 138±12 | 110± 6 | 95± 4 | 111± 5 | 91± 3 |
| 135 | 148± 7 | 140±12 | 109± 6 | 98± 5 | 111± 3 | 91± 3 |
| 150 | 153± 4 | 141±12 | 111± 5 | 100± 5 | 110± 3 | 95± 2 |
| 165 | 155± 5 | 138±12 | 113± 6 | 103± 7 | 112± 4 | 99± 3 |
| 180 | 152± 5 | 141±12 | 120± 8 | 101± 3 | 109± 5 | - |

Die Kombinationen werden hergestellt, indem man entweder die Einzelkomponenten als Pulver intensiv mischt, oder indem man die Einzelkomponenten in einem geeigneten Lösungsmittel wie beispielsweise einem niederen Alkohol auflöst und das Lösungsmittel anschließend entfernt.

Wie oben erwähnt, können die erfindungsgemäßen Kombinationen in Arzneimitteln, besonders zur Behandlung von Bluthochdruck, Herzinsuffizienz und coronarer Herzkrankheit verwendet werden.

Die erfindungsgemäßen Kombinationen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die aktiven Kombinationen und die entsprechenden physiologisch verträglichen Salze kommen z.B. in Frage: Wasser, physiologische Kochsalzlösungen oder Alkohole, z.B. Ethanol, Propandiol, oder Glycerin,

daneben auch Zuckerlösungen wie Glucose- oder Mannitlösungen oder auch eine Mischung aus den verschiedenen genannten Lösungsmitteln.

Als Salze der Verbindungen der Formel I und II kommen, je nach saurer oder basischer Natur dieser Verbindungen, Alkali- oder Erdalkalisalze oder Salze mit physiologisch verträglichen Aminen oder Salze mit anorganischen oder organischen Säuren wie z.B. HCl, HBr, $H_2SO_4$, Maleinsäure, Fumarsäure, Weinsäure, Citronensäure in Frage.

Das folgende Beispiel dient zur Erläuterung der vorliegenden Erfindung, ohne daß diese darauf beschränkt wäre:

Beispiel 1

Herstellung eines oralen Kombinationspräparats aus Ramipril und Chromakalim

1000 Tabletten, die 2 mg Ramipril und 0,3 mg Chromakalim enthalten, werden wie folgt hergestellt:

| | |
|---|---|
| Ramipril | 2 g |
| Chromakalim | 0,3 g |
| Maisstärke | 140 g |
| Gelatine | 7,5 g |
| Mikrokristalline Cellulose | 2,5 g |
| Magnesiumstearat | 2,5 g |

Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 2 mg Ramipril und 0,3 mg Chromakalim enthält.

**Patentansprüche**

**Patentansprüche für folgende Vertragsstaaten : AT, BE, CH, DE, FR, GB, IT, LI, LU, NL, SE**

**1.** Pharmazeutische Zubereitung enthaltend
a) einen ACE-Hemmer der Formel I

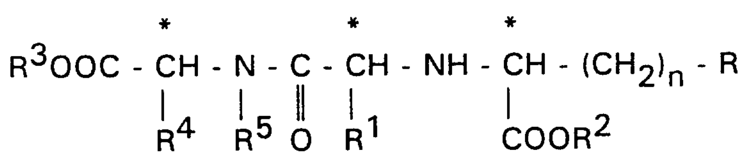

(I)

in welcher
n = 1 oder 2 ist,
R = Waserstoff,
einen gegebenenfalls substituierten aliphatischen Rest mit 1-8 C-Atomen,
einen gegebenenfalls substituierten alicyclischen Rest mit 3-9 C-Atomen,
einen gegebenenfalls substituierten aromatischen Rest mit 6-12 C-Atomen,
einen gegebenenfalls substituierten araliphatischen Rest mit 7-14 C-Atomen,
einen gegebenenfalls substituierten alicyclischaliphatischen Rest mit 7-14 C-Atomen,
einen Rest $OR^a$ oder $SR^a$, worin
$R^a$ für einen gegebenenfalls substituierten aliphatischen Rest mit 1-4 C-Atomen,
für einen gegebenenfalls substituierten aromatischen Rest mit 6-12 C-Atomen oder ein gegebenenfalls substituierten heteroaromatischen Rest mit 5-12 Ringatomen steht,
$R^1$ Wasserstoff,
einen gegebenenfalls substituierten aliphatischen Rest mit 1-6 C-Atomen,
einen gegebenenfalls substituierten alicyclischen Rest mit 3-9 C-Atomen,
einen gegebenenfalls substituierten alicyclischaliphatischen Rest mit 4-13 C-Atomen,

einen gegebenenfalls substituierten aromatischen Rest mit 6-12 C-Atomen,
einen gegebenenfalls substituierten araliphatischen Rest mit 7-16 C-Atomen,
einen gegebenenfalls substituierten heteroaromatischen Rest mit 5-12 Ringatomen oder
die erforderlichenfalls geschützte Seitekette einer natürlich vorkommenden α-Aminosäure bedeuten,
$R^2$ und $R^3$ gleich oder verschieden sind und Wasserstoff, einen gegebenenfalls substituierten aliphatischen Rest mit 1-6 C-Atomen,
einen gegebenenfalls substituierten alicyclischen Rest mit 3-9 C-Atomen,
einen gegebenenfalls substituierten aromatischen Rest mit 6-12 C-Atomen,
einen gegebenenfalls substituierten araliphatischen Rest mit 7-16 C-Atomen bedeuten und
$R^4$ und $R^5$ zusammen mt den diese tragenden Atomen ein Ringsystem aus der Gruppe Tetrahydroisochinolin; Decayhydroisochinolin; Octahydroindol;
Octahydrocyclopenta[b]pyrrol; oder dessen physiologisch verträgliches Salz und
b) einen Kaliumkanal-Modulator der Formel II

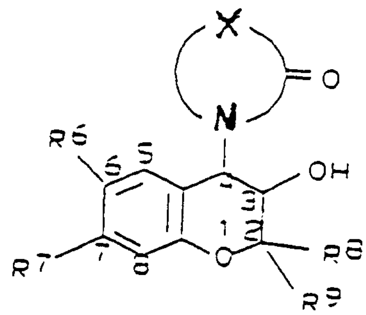

(II)

in welcher
$R^6$ für CN, $NO_2$, $SO_n$-($C_1$-$C_6$)-Alkyl, $SO_n$-Ar, steht; wobei n = 1 oder 2, Ar für ein aromatisches oder heteroaromatisches System steht, das unsubstituiert oder durch 1 bis 3 gleiche oder verschiedene Reste ($C_1$-$C_2$)-Alkyl, ($C_1$-$C_2$)-Alkoxy, Halogen, Trifluormethyl, CN, $NO_2$, CO-($C_1$-$C_2$)-Alkyl, $SO_p$($C_1$-$C_2$) Alkyl, substituiert ist und p für 1 oder 2 steht,
$R^7$ für Wasserstoff, Hydroxy, ($C_1$-$C_2$)-Alkoxy, ($C_1$-$C_2$)-Alkyl, Halogen oder $NR^{10}R^{11}$ steht, wobei $R^{10}$ und $R^{11}$ gleich oder verschieden sind und für Wasserstoff, ($C_1$-$C_2$)Alkyl oder ($C_1$-$C_2$)-Alkylcarbonyl stehen, wobei die oben genannten Bedeutungen von $R^6$ und $R^7$ auch vertauscht sein können,
$R^8$ und $R^9$ gleich oder verschieden sind und für Alkyl mit 1-4 C-Atomen stehen,
X für eine Kette $(CH_2)_m$ steht, die unsubstituiert ist oder durch mindestens 1 und höchstens 2m-1 ($C_1$-$C_2$)-Alkylgruppen substituiert ist, sowie durch ein Heteroatom Y in der Bedeutung von O, $NR^{12}$, S unterbrochen sein kann und $R^{12}$ H oder ($C_1$-$C_4$)-Alkyl bedeutet und m für 2, 3 oder 4 steht, wobei die Konfiguration an $C_3$ und $C_4$ stets entgegengesetzt ist, oder dessen physiologisch verträgliches Salz.

**2.** Zubereitung gemäß Anspruch 1 in welcher im ACE-Hemmer der Formel I
n = 1 oder 2 ist
R Wasserstoff,
Alkyl mit 1-8 C-Atomen,
Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino,Di-($C_1$-$C_4$)-alkylamino, ($C_1$-$C_4$)-Alkanoylamino, Methylendioxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen,

das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann, bedeutet,

$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6-12 C-Atomen, das wie oben bei R beschrieben substituiert sein kann, ($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können,
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürlich vorkommenden $\alpha$-Aminosäure $R^1$-CH($NH_2$)-COOH bedeutet,

$R^2$ und $R^3$ gleich oder verschieden sind und Wasserstoff,
Alkyl mit 1-6 C-Atomen,
Alkenyl mit 2-6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6-12 C-Atomen,
($C_6$-$C_{12}$)-Aryl-($C_1$-$C_4$)-alkyl,
($C_3$-$C_9$)-Cycloalkyl oder
($C_3$-$C_9$)-Cycloalkyl-($C_1$-$C_4$)-alkyl
bedeuten und

$R^4$ und $R^5$ die im Anspruch 1 angegebene Bedeutung haben.

**3.** Zubereitung gemäß einem oder mehreren der Ansprüche 1 oder 2, in welcher im ACE-Hemmer der Formel I

| | |
|---|---|
| n = | 1 oder 2 ist, |
| R | $(C_1\text{-}C_6)$-Alkyl, $(C_2\text{-}C_6)$-Alkenyl, $(C_3\text{-}C_9)$-Cycloalkyl, Amino-$(C_1\text{-}C_4)$-alkyl, $(C_2\text{-}C_5)$-Acylamino-$(C_1\text{-}C_4)$-alkyl, $(C_7\text{-}C_{13})$-Aroylamino-$(C_1\text{-}C_4)$-alkyl, $(C_1\text{-}C_4)$-Alkoxy-carbonylamino-$(C_1\text{-}C_4)$-alkyl, $(C_6\text{-}C_{12})$-Aryl-$(C_1\text{-}C_4)$-alkoxycarbonylamino-$(C_1\text{-}C_4)$-alkyl, $(C_6\text{-}C_{12})$-Aryl, das durch $(C_1\text{-}C_4)$-Alkyl, $(C_1\text{-}C_4)$-Alkoxy, Hydroxy, Halogen, Nitro, Amino, $(C_1\text{-}C_4)$Alkylamino, Di-$(C_1\text{-}C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino- $(C_1\text{-}C_4)$-alkyl, Benzoyloxycarbonylamino-$(C_1\text{-}C_4)$-alkyl oder Phenyl, das durch Phenyl, $(C_1\text{-}C_2)$-Alkyl, ($C_1$ oder $C_2$)-Alkoxy, Hydroxy, Fluor, Chlor, Brom, Amino, $(C_1\text{-}C_4)$-Alkylamino,Di-$(C_1\text{-}C_4)$alkylamino, Nitro und/oder Methylendioxy mono- oder disubstituiert oder im Falle von Methoxy, trisubstituiert sein kann, bedeutet, |
| $R^1$ | Wasserstoff oder $(C_1\text{-}C_6)$-Alkyl, das gegebenenfalls durch Amino, $(C_1\text{-}C_6)$-Acylamino oder Benzoylamino substituiert sein kann, $(C_2\text{-}C_6)$-Alkenyl, $(C_3\text{-}C_9)$-Cycloalkyl, $(C_5\text{-}C_9)$-Cycloalkenyl, $(C_3\text{-}C_7)$-Cycloalkyl-$(C_1\text{-}C_4)$-alkyl, $(C_6\text{-}C_{12})$-Aryl oder teilhydriertes Aryl, das jeweils durch $(C_1\text{-}C_4)$-Alkyl, ($C_1$ oder $C_2$)-Alkoxy oder Halogen substituiert sein kann, $(C_6\text{-}C_{12})$-Aryl-$(C_1\text{-}C_4)$-alkyl oder $(C_7\text{-}C_{13})$-Aroyl-$(C_1\text{-}C_2)$-alkyl, die beide wie vorstehend definiert im Arylrest substituiert sein können, ein mono- bzw. bicyclischer Heterocyclen Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten $\alpha$-Aminosäure, insbesondere aber Wasserstoff, $(C_1\text{-}C_3)$-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet, |
| $R^2$ und $R^3$ | gleiche oder verschiedene Reste Wasserstoff, $(C_1\text{-}C_6)$-Alkyl, $(C_2\text{-}C_6)$-Alkenyl oder $(C_6\text{-}C_{12})$-Aryl-$(C_1\text{-}C_4)$-alkyl, insbesondere aber Wasserstoff, $(C_1\text{-}C_4)$-Alkyl oder Benzyl bedeuten und |
| $R^4$ und $R^5$ | die im Anspruch 1 angegebene Bedeutung haben. |

**4.** Zubereitung gemäß einem oder mehreren der Ansprüche 1 bis 3, enthaltend
2-[N-(1-S-Ethoxycarbonyl-3-phenylpropyl)-S-alanyl]-(1S,3S,5S)-2-azabicyclo[3.3.0]octan-3-carbonsäure
oder deren physiologisch verträgliches Salz.

**5.** Zubereitung gemäß einem oder mehreren der Ansprüche 1 bis 3, enthaltend
1-(N-(1-S-Ethoxycarbonyl-3-phenylpropyl)-S-alanyl]-(2S,3aR,7aS)-octahydro[1H]indol-2-carbonsäure
oder deren physiologisch verträgliches Salz.

**6.** Zubereitung gemäß einem oder mehreren der Ansprüche 1 bis 3, enthaltend
2-[N-(1-S-Ethoxycarbonyl-3-phenylpropyl)-S-alanyl]-1.2.3.4-tetrahydroisochinolin-3-S-carbonsäure
oder deren physiologisch verträgliches Salz.

**7.** Zubereitung gemäß einem oder mehreren der Ansprüche 1 bis 6, enthaltend einen Kaliumkanal-Modulator der Formel II, in welcher
X für eine Kette $(CH_2)_m$ steht; die unsubstituiert ist oder durch eine $(C_1\text{-}C_2)$-Alkylgruppe substituiert ist, sowie durch ein Heteroatom Y, das für O, S oder $NR^{12}$ steht mit $R^{12}$ in der Bedeutung von Wasserstoff oder $(C_2\text{-}C_4)$-Alkyl, unterbrochen sein kann und wobei m für 2, 3 oder 4 steht, oder deren physiologisch verträgliches Salz.

**8.** Zubereitung gemäß einem oder mehreren der Ansprüche 1 bis 6, enthaltend einen Kaliumkanal-Modulator dar Formel II, in welcher,
X für eine Kette $(CH_2)_m$ steht, die unsubstituiert ist oder durch eine $(C_1\text{-}C_2)$-Alkylgruppe substituiert ist wobei m für 3 oder 4 steht,
oder deren physiologisch verträgliches Salz.

**9.** Zubereitung gemäß einem oder mehreren der Ansprüche 1 bis 6, enthaltend einen Kaliumkanal-Modulator der Formel II, in welcher

X für eine Kette $(CH_2)_m$ steht, wobei m für 3 oder 4 steht, die durch eine $(C_1-C_2)$-Alkylgruppe an dem C-Atom substituiert ist, das dem N-Atom des Lactamringes benachbart ist,
oder deren physiologisch verträgliches Salz.

**10.** Zubereitung gemäß einem oder mehreren der Ansprüche 1 bis 6, enthaltend einen Kaliumkanal-Modulator der Formel II, in welcher
X für eine Kette $(CH_2)_m$ steht mit m = 3 oder 4, die unsubstituiert ist oder durch eine $(C_1-C_2)$-Alkylgruppe an dem C-Atom substituiert ist, das dem N-Atom des Lactamringes benachbart ist, und zwar derart, daß die Konfiguration dieses C-Atoms dieselbe ist wie das des C-4 Atom des Chromansystems,
oder deren physiologisch verträgliches Salz.

**11.** Zubereitung gemäß einem oder mehreren der Ansprüche 1 bis 6 , enthaltend einen Kaliumkanal-Modulator der Formel II, in welcher
$R^6$ für $SO_2$-Ar steht mit Ar in der Bedeutung von Phenyl, das unsubstituiert oder durch 1 bis 3 Substituenten wie im Anspruch 8 erwähnt substituiert ist,
$R^7$ für Wasserstoff oder $OCH_3$ steht,
$R^8$ und $R^9$ gleich sind oder verschieden und für $(C_1-C_2)$-Alkyl stehen,
X für eine Kette $(CH_2)_m$ steht mit m = 3 oder 4, die unsubstituiert ist oder durch eine $(C_1-C_2)$-Alkylgruppe an dem C-Atom substituiert ist, das dem N-Atom des Lactamringes benachbart ist, und zwar derart, daß die Konfiguration dieses C-Atoms dieselbe ist wie die des C-4-Atoms im Chromansystems,
oder deren physiologisch verträgliches Salz.

**12.** Zubereitung gemäß einem oder mehreren der Ansprüche 1 bis 6 , enthaltend einen Kaliumkanal-Modulator der Formel II, in welcher
$R^6$ für CN oder $SO_2-CH_3$ und $R^7$ für Wasserstoff,
$R^8$ und $R^9$ gleich oder verschieden sind und für Alkyl mit 1 bis 2 C-Atomen stehen,
X für eine Kette $(CH_2)_m$ steht mit m = 3 oder 4, die unsubstituiert ist oder durch eine $(C_1-C_2)$-Alkylgruppe an dem C-Atom substituiert ist, das dem N-Atom des Lactamringes benachbart ist, und zwar derart, daß die Konfiguration dieses C-Atoms entgegengesetzt ist zu der des C-4 Atoms im Chromansystem,
oder deren physiologisch verträgliches Salz.

**13.** Zubereitung gemäß einem oder mehreren der Ansprüche 1 bis 6 , enthaltend

| | |
|---|---|
| Ramipril + | (±)-6-Cyano-3,4-dihydro-2,2-dimethyl-trans-4-(2-oxo-1-pyrrolidinyl)-2H-benzo[b]-pyran-3-ol (Chromakalim) oder |
| Ramipril + | 6-Cyano-3-hydroxy-2,2-dimethyl-4-(5-methyl-2-oxo-1-pyrrolidinyl)-3,4-dihydro-2H-benzo[b]pyran oder |
| Ramipril + | 2,2-Dimethyl-3-hydroxy-6-phenylsulfonyl-4-(2-oxo-1-pyrrolidinyl)-3,4-dihydro-2H-benzo[b]pyran oder |
| Trandolapril + | Chromakalim oder |
| Trandolapril + | 6-Cyano-3-hydroxy-2,2-dimethyl-4-(5-methyl-2-oxo-1-pyrrolidinyl)-3,4-dihydro-2H-benzo [b]pyran oder |
| Trandolapril + | 2,2-Dimethyl-3-hyroxy-6-phenylsulfonyl-4-(2-oxo-1-pyrrolidinyl)-3,4-dihydro-2H-benzo-[b]pyran oder |
| Quinapril + | Chromakalim oder |
| Quinapril + | 6-Cyano-3-hydroxy-2,2-dimethyl-4-(5-methyl-2-oxo-1-pyrrolidinyl)-3,4-dihydro-2H-benzo[bjpyran oder |
| Quinapril + | 2,2-Dimethyl-3-hydroxy-6-phenylsulfonyl-4-(2-oxo-1-pyrrolidinyl)-3,4-dihydro-2H-benzo[b]pyran oder |
| Ramipril + | (3S,4R)-6-Cyano-3,4-dihydro-2,2-dimethyl-4-(2-oxo-1-pyrrolidinyl)-2H-benzo[b]-pyran-3-ol oder |
| Trandolapril + | (3S,4R)-6-Cyano-3,4-dihydro-2,2-dimethyl-4-(2-oxo-1-pyrrolidinyl)-2H-benzo[b]-pyran-3-ol oder |
| Quinapril + | (3S,4R)-6-Cyano-3,4-dihydro-2,2-dimethyl-4-(2-oxo-1-pyrrolidinyl)-2H-benzo[b]-pyran-3-ol oder |
| Ramipril + | (3S,4R)-6-Phenylsulfonyl-3,4-dihydro-2,2-dimethyl-4-(2-oxo-1-pyrrolidinyl)-2H- |

| | |
|---|---|
| | benzo[b]-pyran-3-ol oder |
| Trandolapril + | (3S,4R)-6-Phenylsulfonyl-3,4-dihydro-2,2-dimethyl-4-(2-oxo-1-pyrrolidinyl)-2H-benzo-[b]pyran-3-ol oder |
| Quinapril + | (3S,4R)-6-Phenylsulfonyl-3,4-dihydro-2,2-dimethyl-4-(2-oxo-1-pyrrolidinyl)-2H-benzo[b]-pyran-3-ol |

sowie jeweils die physiologisch verträglichen Salze der genannten Einzelkomponente, falls solche gebildet werden können.

**14.** Verfahren zur Herstellung einer Zubereitung gemäß einem oder mehreren der Ansprüche 1 bis 13, dadurch gekennzeichnet, daß man
a) einen ACE-Hemmer oder dessen physiologisch verträgliches Salz und
b) einen Kaliumkanal-Modulator oder dessen physiologisch verträgliches Salz
zusammen mit einem physiologisch annehmbaren Träger und gegebenenfalls weiteren Hilfs- und Zusatzstoffen in eine geeignete Darreichungsform bringt.

**15.** Verwendung einer Zubereitung gemäß einem oder mehreren der Ansprüche 1 bis 13 bei der Behandlung des Bluthochdrucks, der Herzinsuffizienz und/oder der coronaren Herzkrankheit.

**16.** Erzeugnis, enthaltend
a) einen ACE-Hemmer der Formel I gemäß Anspruch 1 oder dessen physiologisch verträgliches Salz und
b) einen Kaliumkanal-Modulator der Formel II gemäß Anspruch 1 oder dessen physiologisch verträgliches Salz
als Kombinationspräparat zur gleichzeitigen, getrennten oder zeitlich abgestuften Anwendung bei der Behandlung des Bluthochdrucks.

**Patentansprüche für folgende Vertragsstaaten : ES, GR**

**1.** Verfahren zur Herstellung einer pharmazeutischen Zubereitung enthaltned
a) einen ACE-Hemmer der Formel I

$$R^3OOC-\underset{R^4}{\overset{*}{CH}}-\underset{R^5}{N}-\underset{O}{\overset{}{C}}-\underset{R^1}{\overset{*}{CH}}-NH-\underset{COOR^2}{\overset{*}{CH}}-(CH_2)_n-R \qquad (I)$$

in welcher
n = 1 oder 2 ist,
R = Waserstoff,
einen gegebenenfalls substituierten aliphatischen Rest mit 1-8 C-Atomen,
einen gegebenenfalls substituierten alicyclischen Rest mit 3-9 C-Atomen,
einen gegebenenfalls substituierten aromatischen Rest mit 6-12 C-Atomen,
einen gegebenenfalls substituierten araliphatischen Rest mit 7-14 C-Atomen,
einen gegebenenfalls substituierten alicyclischaliphatischen Rest mit 7-14 C-Atomen,
einen Rest $OR^a$ oder $SR^a$, worin
$R^a$ für einen gegebenenfalls substituierten aliphatischen Rest mit 1-4 C-Atomen,
für einen gegebenenfalls substituierten aromatischen Rest mit 6-12 C-Atomen oder einen gegebenenfalls substituierten heteroaromatischen Rest mit 5-12 Ringatomen steht,
$R^1$ Wasserstoff,
einen gegebenenfalls substituierten aliphatischen Rest mit 1-6 C-Atomen,
einen gegebenenfalls substituierten alicyclischen Rest mit 3-9 C-Atomen,
einen gegebenenfalls substituierten alicyclischaliphatischen Rest mit 4-13 C-Atomen,
einen gegebenenfalls substituierten aromatischen Rest mit 6-12 C-Atomen,
einen gegebenenfalls substituierten araliphatischen Rest mit 7-16 C-Atomen,
einen gegebenenfalls substituierten heteroaromatischen Rest mit 5-12 Ringatomen oder
die erforderlichenfalls geschützte Seitekette einer natürlich vorkommenden α-Aminosäure bedeuten,

$R^2$ und $R^3$ gleich oder verschieden sind und Wasserstoff, einen gegebenenfalls substituierten aliphatischen Rest mit 1-6 C-Atomen,
einen gegebenenfalls substituierten alicyclischen Rest mit 3-9 C-Atomen,
einen gegebenenfalls substituierten aromatischen Rest mit 6-12 C-Atomen,
einen gegebenenfalls substituierten araliphatischen Rest mit 7-16 C-Atomen bedeuten und
$R^4$ und $R^5$ zusammen mt den diese tragenden Atomen ein Ringsystem aus der Gruppe Tetrahydroisochinolin; Decayhydroisochinolin; Octahydroindol; Octahydrocyclopenta[b]pyrrol; oder dessen physiologisch verträgliches Salz und
b) einen Kaliumkanal-Modulator der Formel II

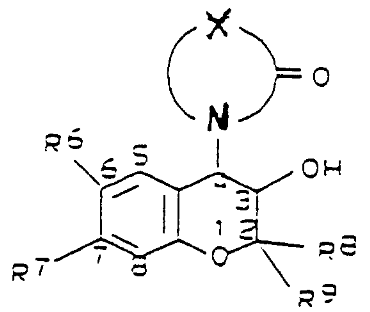

(II)

in welcher
$R^6$ für CN, $NO_2$, $SO_n$-($C_1$-$C_6$)-Alkyl, $SO_n$-Ar, steht; wobei n = 1 oder 2, Ar für ein aromatisches oder heteroaromatisches System steht, das unsubstituiert oder durch 1 bis 3 gleiche oder verschiedene Reste ($C_1$-$C_2$)-Alkyl, ($C_1$-$C_2$)-Alkoxy, Halogen, Trifluormethyl, CN, $NO_2$, CO-($C_1$-$C_2$)-Alkyl, $SO_p$-($C^1$-$C_2$) Alkyl, substituiert ist und p für 1 oder 2 steht,
$R^7$ für Wasserstoff, Hydroxy, ($C_1$-$C_2$)-Alkoxy, ($C_1$-$C_2$]-Alkyl, Halogen oder $NR^{10}R^{11}$ steht, wobei $R^{10}$ und $R^{11}$ gleich oder verschieden sind und für Wasserstoff, ($C_1$-$C_2$)Alkyl oder ($C_1$-$C_2$)-Alkylcarbonyl stehen, wobei die oben genannten Bedeutungen von $R^6$ und $R^7$ auch vertauscht sein können,
$R^8$ und $R^9$ gleich oder verschieden sind und für Alkyl mit 1-4 C-Atomen stehen,
X für eine Kette $(CH_2)_m$ steht, die unsubstituiert ist oder durch mindestens 1 und höchstens 2m-1 ($C_1$-$C_2$)-Alkylgruppen substituiert ist, sowie durch ein Heteroatom Y in der Bedeutung von O, $NR^{12}$, S unterbrochen sein kann und $R^{12}$ H oder ($C_1$-$C_4$)-Alkyl bedeutet und m für 2, 3 oder 4 steht, wobei die Konfiguration an $C_3$ und $C_4$ stets entgegengesetzt ist, oder dessen physiologisch verträgliches Salz,
dadurch gekennzeichnet, daß man
a) einen ACE-Hemmer oder dessen physiologisch verträgliches Salz und
b) einen Kaliumkanal-Modulator oder dessen physiologisch verträgliches Salz
zusammen mit einem physiologisch annehmbaren Träger und gegebenenfalls weiteren Hilfs- und Zusatzstoffen in eine geeignete Darreichungsform bringt.

**2.** Verfahren gemäß Anspruch 1 , dadurch gekennzeichnet, daß im ACE-Hemmer der Formel I
n = 1 oder 2 ist
R Wasserstoff,
Alkyl mit 1-8 C-Atomen,
Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino,Di-($C_1$-$C_4$)-alkylamino, ($C_1$-$C_4$)-Alkanoylamino, Methylendioxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen,

das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($c_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann, bedeutet,

$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6-12 C-Atomen, das wie oben bei R beschrieben substituiert sein kann, ($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können,
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürlich vorkommenden $\alpha$-Aminosäure $R^1$-CH($NH_2$)-COOH bedeutet,

$R^2$ und $R^3$ gleich oder verschieden sind und Wasserstoff,
Alkyl mit 1-6 C-Atomen,
Alkenyl mit 2-6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6-12 C-Atomen,
($C_6$-$C_{12}$)-Aryl-($C_1$-$C_4$)-alkyl,
($C_3$-$C_9$)-Cycloalkyl oder
($C_3$-$C_9$)-Cycloalkyl-($C_1$-$C_4$)-alkyl
bedeuten und

$R^4$ und $R^5$ die im Anspruch 1 angegebene Bedeutung haben.

**3.** Verfahren gemäß einem oder mehreren der Ansprüche 1 oder 2 bis 3, dadurch gekennzeichnet, daß im ACE-Hemmer der Formel I

n = 1 oder 2 ist,

R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert. Butoxycarbonylamino- ($C_1$-$C_4$)-alkyl, Benzoyloxycarbonylamino-($C_1$-$C_4$)-alkyl oder Phenyl, das durch Phenyl, ($C_1$-$C_2$)-Alkyl, ($C_1$ oder $C_2$)-Alkoxy, Hydroxy, Fluor, Chlor, Brom, Amino, ($C_1$-$C_4$)-Alkylamino,Di-($C_1$-$C_4$)alkylamino, Nitro und/oder Methylendioxy mono- oder disubstituiert oder im Falle von Methoxy, trisubstituiert sein kann, bedeutet,

$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$-$C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Aminobutyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl ($C_1$-$C_4$)-alkyl, insbesondere aber Wasserstoff, ($C_1$-$C_4$)-Alkyl oder Benzyl bedeuten und

$R^4$ und $R^5$ die im Anspruch 1 angegebene Bedeutung haben.

**4.** Verfahren gemäß einem oder mehreren der Ansprüche 1 bis 3, dadurch gekennzeichnet, daß die Zubereitung 2-[N-(1-S-Ethoxycarbonyl-3-phenylpropyl)-S-alanyl]-(1S,3S,5S)-2-azabicyclo[3.3.0]octan-3-carbonsäure oder ein physiologisch verträgliches Salz davon enthält.

**5.** Verfahren gemäß einem oder mehreren der Ansprüche 1 bis 3, dadurch gekennzeichnet, daß die Zubereitung 1-[N-(1-S-Ethoxycarbonyl-3-phenylpropyl)-S-alanyl]-(2S,3aR,7aS)-octahydro[1H]indol-2-carbonsäure oder ein physiologisch verträgliches Salz davon enthält.

**6.** Verfahren gemäß einem oder mehreren der Ansprüche 1 bis 3, dadurch gekennzeichnet, daß die Zubereitung 2-[N-(1-S-Ethoxycarbonyl-3-phenylpropyl)-S-alanyl]-1.2.3.4-tetrahydroisochinolin-3-S-carbonsäure oder ein physiologisch verträgliches Salz davon enthält.

**7.** Verfahren gemäß einem oder mehreren der Ansprüche 1 bis 6, dadurch gekennzeichnet, daß die Zubereitung einen Kaliumkanal-Modulator der Formel II, in welcher
X für eine Kette $(CH_2)_m$ steht; die unsubstituiert ist oder durch eine ($C_1$-$C_2$)-Alkylgruppe substituiert ist, sowie durch ein Heteroatom Y, das für O, S oder $NR^{12}$ steht mit $R^{12}$ in der Bedeutung von Wasserstoff oder ($C_2$-$C_4$)-Alkyl, unterbrochen sein kann und wobei m für 2, 3 oder 4 steht, oder ein physiologisch verträgliches Salz davon enthält.

**8.** Verfahren gemäß einem oder mehreren der Ansprüche 1 bis 6, dadurch gekennzeichnet, daß die Zubereitung einen Kaliumkanal-Modulator der Formel II, in welcher,
X für eine Kette $(CH_2)_m$ steht, die unsubstituiert ist oder durch eine ($C_1$-$C_2$)-Alkylgruppe substituiert ist wobei m für 3 oder 4 steht,
oder ein physiologisch verträgliches Salz davon enthält.

**9.** Verfahren gemäß einem oder mehreren der Ansprüche 1 bis 6, dadurch gekennzeichnet, daß die Zubereitung einen Kaliumkanal-Modulator der Formel II, in welcher

X für eine Kette $(CH_2)_m$ steht, wobei m für 3 oder 4 steht, die durch eine $(C_1$-$C_2)$-Alkylgruppe an dem C-Atom substituiert ist, das dem N-Atom des Lactamringes benachbart ist,
oder ein physiologisch verträgliches Salz davon, enthält.

**10.** Verfahren gemäß einem oder mehreren der Ansprüche 1 bis 6, dadurch gekennzeichnet, daß die Zubereitung einen Kaliumkanal-Modulator der Formel II, in welcher
X für eine Kette $(CH_2)_m$ steht mit m = 3 oder 4, die unsubstituiert ist oder durch eine $(C_1$-$C_2)$-Alkylgruppe an dem C-Atom substituiert ist, das dem N-Atom des Lactamringes benachbart ist, und zwar derart, daß die Konfiguration dieses C-Atoms dieselbe ist wie das des C-4 Atom des Chromansystems,
oder ein physiologisch verträgliches Salz davon, enthält.

**11.** Verfahren gemäß einem oder mehreren der Ansprüche 1 bis 10, dadurch gekennzeichnet, daß die Zubereitung einen Kaliumkanal-Modulator der Formel II, in welcher
$R^6$ für $SO_2$-Ar steht mit Ar in der Bedeutung von Phenyl, das unsubstituiert oder durch 1 bis 3 Substituenten wie im Anspruch 8 erwähnt substituiert ist,
$R^7$ für Wasserstoff oder $OCH_3$ steht,
$R^8$ und $R^9$ gleich sind oder verschieden und für $(C_1$-$C_2)$-Alkyl stehen,
X für eine Kette $(CH_2)_m$ steht mit m = 3 oder 4, die unsubstituiert ist oder durch eine $(C_1$-$C_2)$-Alkylgruppe an dem C-Atom substituiert ist, das dem N-Atom des Lactamringes benachbart ist, und zwar derart, daß die Konfiguration dieses C-Atoms dieselbe ist wie die des C-4-Atoms im Chromansystems,
oder ein physiologisch verträgliches Salz davon, enthält.

**12.** Verfahren gemäß einem oder mehreren der Ansprüche 1 bis 11, dadurch gekennzeichnet, daß die Zubereitung einen Kaliumkanal-Modulator der Formel II, in welcher
$R^6$ für CN oder $SO_2$-$CH_3$ und $R^7$ für Wasserstoff,
$R^8$ und $R^9$ gleich oder verschieden sind und für Alkyl mit 1 bis 2 C-Atomen stehen,
X für eine Kette $(CH_2)_m$ steht mit m = 3 oder 4, die unsubstituiert ist oder durch eine $(C_1$-$C_2)$-Alkylgruppe an dem C-Atom substituiert ist, das dem N-Atom des Lactamringes benachbart ist, und zwar derart, daß die Konfiguration dieses C-Atoms entgegengesetzt ist zu der des C-4 Atoms im Chromansystem,
oder ein physiologisch verträgliches Salz davon, enthält.

**13.** Verfahren gemäß einem oder mehreren der Ansprüche 1 bis 12, dadurch gekennzeichnet, daß die Zubereitung

| | |
|---|---|
| Ramipril + | (±)-6-Cyano-3,4-dihydro-2,2-dimethyl-trans-4-(2-oxo-1-pyrrolidinyl)-2H-benzo[b]-pyran-3-ol (Chromakalim) oder |
| Ramipril + | 6-Cyano-3-hydroxy-2,2-dimethyl-4-(5-methyl-2-oxo-1-pyrrolidinyl)-3-4-dihydro-2H-benzo[b]pyran oder |
| Ramipril + | 2,2-Dimethyl-3-hydroxy-6-phenylsulfonyl-4-(2-oxo-1-pyrrolidinyl)-3,4-dihydro-2H-benzo[b]pyran oder |
| Trandolapril + | Chromakalim oder |
| Trandolapril + | 6-Cyano-3-hydroxy-2,2-dimethyl-4-(5-methyl-2-oxo-1-pyrrolidinyl)-3,4-dihydro-2H-benzo [b]pyran oder |
| Trandolapril + | 2,2-Dimethyl-3-hyroxy-6-phenylsulfonyl-4-(2-oxo-1-pyrrolidinyl)-3,4-dihydro-2H-benzo-[b]pyran oder |
| Quinapril + | Chromakalim oder |
| Quinapril + | 6-Cyano-3-hydroxy-2,2-dimethyl-4-(5-methyl-2-oxo-1-pyrrolidinyl)-3,4-dihydro-2H-benzo[b]pyran oder |
| Quinapril + | 2,2-Dimethyl-3-hydroxy-6-phenylsulfonyl-4-(2-oxo-1-pyrrolidinyl)-3,4-dihydro-2H-benzo[b]pyran oder |
| Ramipril + | (3S,4R)-6-Cyano-3,4-dihydro-2,2-dimethyl-4-(2-oxo-1-pyrrolidinyl)-2H-benzo[b]-pyran-3-ol oder |
| Trandolapril + | (3S,4R)-6-Cyano-3,4-dihydro-2,2-dimethyl-4-(2-oxo-1-pyrrolidinyl)-2H-benzo[b]-pyran-3-ol oder |
| Quinapril + | (3S,4R)-6-Cyano-3,4-dihydro-2,2-dimethyl-4-(2-oxo-1-pyrrolidinyl)-2H-benzo[b]-pyran-3-ol oder |

| | |
|---|---|
| Ramipril + | (3S,4R)-6-Phenylsulfonyl-3,4-dihydro-2,2-dimethyl-4-(2-oxo-1-pyrrolidinyl)-2H-benzo[b]-pyran-3-ol oder |
| Trandolapril + | (3S,4R)-6-Phenylsulfonyl-3,4-dihydro-2,2-dimethyl-4-(2-oxo-1-pyrrolidinyl)-2H-benzo-[b]pyran-3-ol oder |
| Quinapril + | (3S,4R)-6-Phenylsulfonyl-3,4-dihydro-2,2-dimethyl-4-(2-oxo-1-pyrrolidinyl)-2H-benzo[b]-pyran-3-ol |

sowie jeweils die physiologisch verträglichen Salze der genannten Einzelkomponente, falls solche gebildet werden können, enthält.

**14.** Verwendung einer Zubereitung gemäß einem oder mehreren der Ansprüche 1 bis 13 bei der Behandlung des Bluthochdrucks, der Herzinsuffizienz und/oder der coronaren Herzkrankheit.

**15.** Verfahren zur Herstellung eines Erzeugnisses, enthaltend
a) einen ACE-Hemmer der Formel I gemäß Anspruch 1 oder dessen physiologisch verträgliches Salz
b) einen Kaliumkanal-Modulator der Formel II gemäß Anspruch 1 oder dessen physiologisch verträgliches Salz
als Kombinationspräparat zur gleichzeitigen, getrennten oder zeitlich abgestuften Anwendung bei der Behandlung des Bluthochdrucks, dadurch gekennzeichnet, daß
a) einen ACE-Hemmer oder dessen physiologisch verträgliches Salz und
b) einen Kaliumkanal-Modulator oder dessen physiologisch verträgliches Salz
zusammen mit einem physiologisch annehmbaren Träger und gegebenenfalls weiteren Hilfs- und Zusatzstoffen in eine geeignete Darreichungsform bringt.

## Claims

**Claims for the following Contracting States : AT, BE, CH, DE, FR, GB, IT, LI, LU, NL, SE**

**1.** A pharmaceutical preparation containing
a) an ACE inhibitor of the formula I

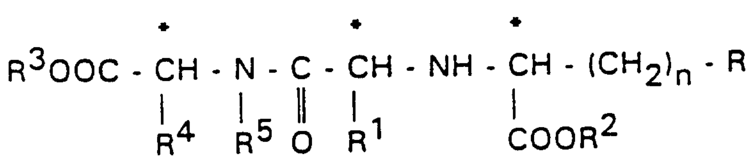

(I)

in which

| | |
|---|---|
| n | is 1 or 2, |
| R | denotes hydrogen, an optionally substituted aliphatic radical having 1-8 carbon atoms, an optionally substituted alicyclic radical having 3-9 carbon atoms, an optionally substituted aromatic radical having 6-12 carbon atoms, an optionally substituted araliphatic radical having 7-14 carbon atoms, an optionally substituted alicyclic-aliphatic radical having 7-14 carbon atoms, a radical $OR^a$ or $SR^a$, in which |
| $R^a$ | represents an optionally substituted aliphatic radical having 1-4 carbon atoms, an optionally substituted aromatic radical having 6-12 carbon atoms or an optionally substituted heteroaromatic radical having 5-12 ring atoms, |
| $R^1$ | denotes hydrogen, an optionally substituted aliphatic radical having 1-6 carbon atoms, an optionally substituted alicyclic radical having 3-9 carbon atoms, an optionally substituted alicyclic-aliphatic radical having 4-13 carbon atoms, an optionally substituted aromatic radical having 6-12 carbon atoms, an optionally substituted araliphatic radical having 7-16 carbon atoms, an optionally substituted heteroaromatic radical having 5-12 ring atoms or the side chain, protected if necessary, of a naturally occurring $\alpha$-amino acid, |
| $R^2$ and $R^3$ | are identical or different and denote hydrogen, |

an optionally substituted aliphatic radical having 1-6 carbon atoms,
an optionally substituted alicyclic radical having 3-9 carbon atoms,
an optionally substituted aromatic radical having 6-12 carbon atoms,
an optionally substituted araliphatic radical having 7-16 carbon atoms and

$R^4$ and $R^5$ together with the atoms carrying them form a ring system from the group comprising tetrahydroisoquinoline, decahydroisoquinoline, octahydroindole and octahydrocyclopenta[b]-pyrrole,

or its physiologically tolerable salt
and
b) a potassium channel modulator of the formula II

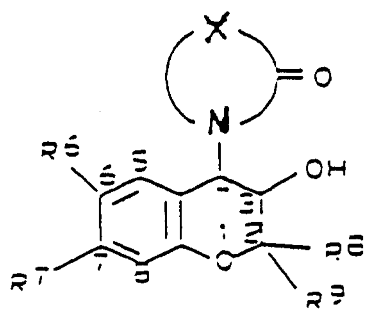

(II)

in which

$R^6$ represents CN, $NO_2$, $SO_n$-($C_1$-$C_6$)-alkyl or $SO_n$-Ar, where n is 1 or 2, Ar represents an aromatic or heteroaromatic system which is unsubstituted or substituted by 1 to 3 identical or different radicals from the series comprising ($C_1$-$C_2$)-alkyl, ($C_1$-$C_2$)-alkoxy, halogen, trifluoromethyl, CN, $NO_2$, CO-($C_1$-$C_2$)-alkyl or $SO_p$-($C_1$-$C_2$)-alkyl and p represents 1 or 2,

$R^7$ represents hydrogen, hydroxyl, ($C_1$-$C_2$)-alkoxy, ($C_1$-$C_2$)-alkyl, halogen or $NR^{10}R^{11}$, where $R^{10}$ and $R^{11}$ are identical or different and represent hydrogen, ($C_1$-$C_2$)-alkyl or ($C_1$-$C_2$)-alkylcarbonyl, where the abovementioned meanings of $R^6$ and $R^7$ can also be interchanged,

$R^8$ and $R^9$ are identical or different and represent alkyl having 1-4 carbon atoms,

X represents a $(CE_2)_m$ chain which is unsubstituted or substituted by at least 1 and at most 2m-1 ($C_1$-$C_2$)-alkyl groups, and can be interrupted by a hetero- atom Y with the meaning of O, $NR^{12}$ or S and $R^{12}$ denotes H or ($C_1$-$C_4$)-alkyl and m represents 2, 3 or 4, where the configuration of $C_3$ and $C_4$ is always opposite,

or its physiologically tolerable salt.

**2.** The preparation as claimed in claim 1, in which in the ACE inhibitor of the formula I

n is 1 or 2

R denotes hydrogen,
alkyl having 1-8 carbon atoms,
alkenyl having 2-6 carbon atoms,
cycloalkyl having 3-9 carbon atoms,
aryl having 6-12 carbon atoms,
which can be monosubstituted, disubstituted or trisubstituted by ($C_1$-$C_4$)-alkyl, ($C_1$-$C_4$)-alkoxy, hydroxyl, halogen, nitro, amino, aminomethyl, ($C_1$-$C_4$)-alkyl-amino, di-($C_1$-$C_4$)-alkylamino, ($C_1$-$C_4$)-alkanoylamino, methylenedioxy, cyano and/or sulfamoyl,
alkoxy having 1-4 carbon atoms,
aryloxy having 6-12 carbon atoms,
which can be substituted as described above for aryl,
mono- or bicyclic heteroaryloxy having 5-7 or 8-10 ring atoms, of which 1 or 2 ring atoms are sulfur or oxygen atoms and/or 1 to 4 ring atoms are nitrogen,
which can be substituted as described above for aryl,
amino-($C_1$-$C_4$)-alkyl,
($C_1$-$C_4$)-alkanoylamino-($C_1$-$C_4$)-alkyl,

$(C_7\text{-}C_{13})$-aroylamino-$(C_1\text{-}C_4)$-alkyl,
$(C_1\text{-}C_4)$-alkoxycarbonylamino-$(C_1\text{-}C_4)$-alkyl,
$(C_6\text{-}C_{12})$-aryl-$(C_1\text{-}C_4)$-alkoxycarbonylamino-$(C_1\text{-}C_4)$-alkyl,
$(C_6\text{-}C_{12})$-aryl-$(C_1\text{-}C_4)$-alkylamino-$(C_1\text{-}C_4)$-alkyl,
$(C_1\text{-}C_4)$-alkylamino-$(C_1\text{-}C_4)$-alkyl,
di-$(C_1\text{-}C_4)$-alkylamino-$(C_1\text{-}C_4)$-alkyl,
guanidino-$(C_1\text{-}C_4)$-alkyl,
imidazolyl, indolyl,
$(C_1\text{-}C_4)$-alkylthio,
$(C_1\text{-}C_4)$-alkylthio-$(C_1\text{-}C_4)$-alkyl,
$(C_6\text{-}C_{12})$-arylthio-$(C_1\text{-}C_4)$-alkyl,
which can be substituted in the aryl moiety as described above for aryl,
$(C_6\text{-}C_{12})$-aryl-$(C_1\text{-}C_4)$-alkylthio,
which can be substituted in the aryl moiety as described above for aryl,
carboxyl-$(C_1\text{-}C_4)$-alkyl,
carboxyl, carbamoyl,
carbamoyl-$(C_1\text{-}C_4)$-alkyl,
$(C_1\text{-}C_4)$-alkoxycarbonyl-$(C_1\text{-}C_4)$-alkyl,
$(C_6\text{-}C_{12})$-aryloxy-$(C_1\text{-}C_4)$-alkyl,
which can be substituted in the aryl moiety as described above for aryl or
$(C_6\text{-}C_{12})$-aryl-$(C_1\text{-}C_4)$-alkoxy,
which can be substituted in the aryl moiety as described above for aryl,

$R^1$ denotes hydrogen,
alkyl having 1-6 carbon atoms,
alkenyl having 2-6 carbon atoms,
alkynyl having 2-6 carbon atoms,
cycloalkyl having 3-9 carbon atoms,
cycloalkenyl having 5-9 carbon atoms,
$(C_3\text{-}C_9)$-cycloalkyl-$(C_1\text{-}C_4)$-alkyl,
$(C_5\text{-}C_9)$-cycloalkenyl-$(C_1\text{-}C_4)$-alkyl,
aryl, which is optionally partly hydrogenated, having 6-12 carbon atoms, which can be substituted as described above for R,
$(C_6\text{-}C_{12})$-aryl-$(C_1\text{-}C_4)$-alkyl or $(C_7\text{-}C_{13})$-aroyl-($C_1$ or $C_2$)-alkyl
which can both be substituted like the above-mentioned aryl,
mono- or bicyclic heteroaryl, which is optionally partly hydrogenated, having 5-7 or 8-10 ring atoms, of which 1 or 2 ring atoms are sulfur or oxygen atoms and/or 1 to 4 ring atoms are nitrogen atoms,
which can be substituted like the above-mentioned aryl or
the optionally protected side chain of a naturally occurring $\alpha$-amino acid $R^1$-$CH(NH_2)$-COOH,

$R^2$ and $R^3$ are identical or different and denote hydrogen,
alkyl having 1-6 carbon atoms,
alkenyl having 2-6 carbon atoms,
di-$(C_1\text{-}C_4)$-alkylamino-$(C_1\text{-}C_4)$-alkyl,
$(C_1\text{-}C_5)$-alkanoyloxy-$(C_1\text{-}C_4)$-alkyl,
$(C_1\text{-}C_6)$-alkoxycarbonylory-$(C_1\text{-}C_4)$-alkyl,
$(C_7\text{-}C_{13})$-aroyloxy$(C_1\text{-}C_4)$-alkyl,
$(C_6\text{-}C_{12})$-aryloxycarbonyloxy-$(C_1\text{-}C_4)$-alkyl,
aryl having 6-12 carbon atoms,
$(C_6\text{-}C_{12})$-aryl-$(C_1\text{-}C_4)$-alkyl,
$(C_3\text{-}C_9)$-cycloalkyl or
$(C_3\text{-}C_9)$-cycloalkyl-$(C_1\text{-}C_4)$-alkyl
and

$R^4$ and $R^5$ have the meaning indicated in claim 1.

**3.** The preparation as claimed in one or more of claims 1 and 2, in which in the ACE inhibitor of the formula I

n is 1 or 2,

R denotes $(C_1-C_6)$-alkyl, $(C_2-C_6)$-alkenyl, $(C_3-C_9)$-cycloalkyl, amino-$(C_1-C_4)$-alkyl, $(C_2-C_5)$-acylamino-$(C_1-C_4)$-alkyl, $(C_7-C_{13})$-aroylamino-$(C_1-C_4)$-alkyl, $(C_1-C_4)$-alkoxycarbonylamino- $(C_1-C_4)$-alkyl, $(C_6-C_{12})$-aryl-$(C_2-C_4)$-alkoxycarbonylamino-$(C_1-C_4)$-alkyl, $(C_6-C_{12})$-aryl which can be monosubstituted, disubstituted or trisubstituted by $(C_1-C_4)$-alkyl, $(C_1-C_4)$-alkoxy, hydroxyl, halogen, nitro, amino, $(C_1-C_4)$-alkylamino, di-$(C_1-C_4)$-alkylamino and/or methylenedioxy, or 3-indolyl, in particular methyl, ethyl, cyclohexyl, tert.-butoxycarbonylamino-$(C_1-C_4)$-alkyl, benzoyloxycarbonylamino-$(C_1-C_4)$-alkyl or phenyl which can be monosubstituted or disubstituted by phenyl, $(C_1-C_2)$-alkyl, ($C_1$ or $C_2$)-alkoxy, hydroxyl, fluorine, chlorine, bromine, amino, $(C_1-C_4)$-alkylamino, di-$(C_1-C_4)$-alkylamino, nitro and/or methylenedioxy or, in the case of methoxy, trisubstituted,

$R^1$ denotes hydrogen or $(C_1-C_6)$-alkyl which can optionally be substituted by amino, $(C_1-C_6)$-acylamino or benzoylamino, $(C_2-C_6)$-alkenyl, $(C_3-C_9)$-cycloalkyl, $(C_5-C_9)$-cycloalkenyl, $(C_3-C_7)$-cycloalkyl-$(C_1-C_4)$-alkyl, $(C_6-C_{12})$-aryl or partly hydrogenated aryl, which in each case can be substituted by $(C_1-C_4)$-alkyl, ($C_1$ or $C_2$)-alkoxy or halogen, $(C_6-C_{12})$-aryl-$(C_1-C_4)$-alkyl or $(C_7-C_{13})$-aroyl-$(C_1-C_2)$-alkyl, which can both be substituted in the aryl radical as defined in the foregoing, a mono- or bicyclic heterocyclic radical having 5 to 7 or 8 to 10 ring atoms, of which 1 or 2 ring atoms are sulfur or oxygen atoms and/or 1 to 4 ring atoms are nitrogen atoms, or a side chain of a naturally occurring, optionally protected $\alpha$-amino acid, but in particular hydrogen, $(C_1-C_3)$-alkyl, ($C_2$ or $C_3$)-alkenyl, the optionally protected side chain of lysine, benzyl, 4-methoxybenzyl, 4-ethoxybenzyl, phenethyl, 4-aminobutyl or benzoylmethyl,

$R^2$ and $R^3$ denote identical or different radicals hydrogen, $(C_1-C_6)$-alkyl, $(C_2-C_6)$-alkenyl or $(C_6-C_{12})$-aryl-$(C_1-C_4)$-alkyl, but in particular hydrogen, $(C_1-C_4)$-alkyl or benzyl and

$R^4$ and $R^5$ have the meaning indicated in claim 1 or 2.

**4.** The preparation as claimed in one or more of claims 1 to 3, containing
2-[N-(1-S-ethoxycarbonyl-3-phenylpropyl)-S-alanyl]-(1S,3S,5S)-2-azabicyclo[3.3.0]octane-3-carboxylic acid
or its physiologically tolerable salt.

**5.** The preparation as claimed in one or more of claims 1 to 3, containing
1-[N-(1-S-ethoxycarbonyl-3-phenylpropyl)-S-alanyl]-(2S,3aR,7aS)-octahydro[1H]indole-2-carboxylic acid
or its physiologically tolerable salt.

**6.** The preparation as claimed in one or more of claims 1 to 3, containing
2-[N-(1-S-ethoxycarbonyl-3-phenylpropyl)-S-alanyl]-1,2,3,4-tetrahydroisoquinoline-3-S-carboxylic acid
or its physiologically tolerable salt.

**7.** The preparation as claimed in one or more of claims 1 to 6, containing a potassium channel modulator of the formula II in which
X represents a $(CH_2)_m$ chain which is unsubstituted or substituted by a $(C_1-C_2)$-alkyl group, and can be interrupted by a heteroatom Y which represents O, S or $NR^{12}$ with $R^{12}$ having the meaning of hydrogen or $(C_2-C_4)$-alkyl, and where m represents 2, 3 or 4,
or its physiologically tolerable salt.

**8.** The preparation as claimed in one or more of claims 1 to 6, containing a potassium channel modulator of the formula II in which
X represents a $(CH_2)_m$ chain which is unsubstituted or substituted by a $(C_1-C_2)$-alkyl group where m represents 3 or 4,
or its physiologically tolerable salt.

**9.** The preparation as claimed in one or more of claims 1 to 6, containing a potassium channel modulator of the formula II in which
X represents a $(CH_2)_m$ chain, where m represents 3 or 4, which is substituted by a $(C_1-C_2)$-alkyl group on the carbon atom which is adjacent to the nitrogen atom of the lactam ring,
or its physiologically tolerable salt.

**10.** The preparation as claimed in one or more of claims 1 to 6, containing a potassium channel modulator of the formula II in which
X represents a $(CH_2)_m$ chain having m = 3 or 4 which is unsubstituted or substituted by a $(C_1-C_2)$-alkyl group on the carbon atom which is adjacent to the nitrogen atom of the lactam ring, in particular in such a way that the configuration of this carbon atom is the same as that of the 4-carbon atom of the chroman system,
or its physiologically tolerable salt.

**11.** The preparation as claimed in one or more of claims 1 to 6, containing a potassium channel modulator of the formula II in which
$R^6$ represents $SO_2$-Ar with Ar having the meaning of phenyl which is unsubstituted or substituted by 1 to 3 substituents as mentioned in claim 8,
$R^7$ represents hydrogen or $OCH_3$,
$R^8$ and $R^9$ are identical or different and represent $(C_1-C_2)$-alkyl,
X represents a $(CH_2)_m$ chain having m = 3 or 4 which is unsubstituted or substituted by a $(C_1-C_2)$-alkyl group on the carbon atom which is adjacent to the nitrogen atom of the lactam ring, in particular in such a way that the configuration of this carbon atom is the same as that of the 4-carbon atom in the chroman system,
or its physiologically tolerable salt.

**12.** The preparation as claimed in one or more of claims 1 to 6, containing a potassium channel modulator of the formula II in which
$R^6$ represents CN or $SO_2-CH_3$ and $R^7$ represents hydrogen,
$R^8$ and $R^9$ are identical or different and represent alkyl having 1 or 2 carbon atoms,
X represents a $(CH_2)_m$ chain having m = 3 or 4 which is unsubstituted or substituted by a $(C_1-C_2)$-alkyl group on the carbon atom which is adjacent to the nitrogen atom of the lactam ring, in particular in such a way that the configuration of this carbon atom is opposite to that of the 4-carbon atom in the chroman system,
or its physiologically tolerable salt.

**13.** The preparation as claimed in one or more of claims 1 to 6, containing

| | |
|---|---|
| ramipril + | (±)-6-cyano-3,4-dihydro-2,2-dimethyl-trans-4-(2-oxo-1-pyrrolidinyl)-2H-benzo[b]-pyran-3-ol (chromakalim) or, |
| ramipril + | 6-cyano-3-hydroxy-2,2-dimethyl-4-(5-methyl-2-oxo-1-pyrrolidinyl)-3,4-dihydro-2H-benzo[b]pyran or |
| ramipril + | 2,2-dimethyl-3-hydroxy-6-phenylsulfonyl-4-(2-oxo-1-pyrrolidinyl)-3,4-dihydro-2H-benzo[b]pyran or |
| trandolapril + | chromakalim or |
| trandolapril + | 6-cyano-3-hydroxy-2,2-dimethyl-4-(5-methyl-2-oxo-1-pyrrolidinyl)-3,4-dihydro-2H-benzo[b]pyran or |
| trandolapril + | 2,2-dimethyl-3-hydroxy-6-phenylsulfonyl-4-(2-oxo-1-pyrrolidinyl)-3,4-dihydro-2H-benzo[b]pyran or |
| quinapril + | chromakalim or |
| quinapril + | 6-cyano-3-hydroxy-2,2-dimethyl-4-(5-methyl-2-oxo-1-pyrrolidinyl)-3,4-dihydro-2H-benzo[b]pyran or |
| quinapril + | 2,2-dimethyl-3-hydroxy-6-phenylsulfonyl-4-(2-oxo-1-pyrrolidinyl)-3,4-dihydro-2H-benzo-[b]pyran or |
| ramipril + | (3S,4R)-6-cyano-3,4-dihydro-2,2-dimethyl-4-(2-oxo-1-pyrrolidinyl)-2H-benzo[b]-pyran-3-ol or |
| trandolapril + | (3S,4R)-6-cyano-3,4-dihydro-2,2-dimethyl-4-(2-oxo-1-pyrrolidinyl)-2H-benzo[b]-pyran-3-ol or |
| quinapril + | (3S,4R)-6-cyano-3,4-dihydro-2,2-dimethyl-4-(2-oxo-1-pyrrolidinyl)-2H-benzo[b]-pyran-3-ol or |
| ramipril + | (3S,4R)-6-phenylsulfonyl-3,4-dihydro-2,2-dimethyl-4-(2-oxo-1-pyrrolidinyl)-2H-benzo[b]-pyran-3-ol or |
| trandolapril + | (3S,4R)-6-phenylsulfonyl-3,4-dihydro-2,2-dimethyl-4-(2-oxo-1-pyrrolidinyl)-2H-benzo[b]pyran-3-ol or |
| quinapril + | (3S,4R)-6-phenylsulfonyl-3,4-dihydro-2,2-dimethyl-4-(2-oxo-1-pyrrolidinyl)-2H- |

benzo[b]pyran-3-ol

and in each case the physiologically tolerable salts of the individual components mentioned, if these can be formed.

**14.** The process for the preparation of a preparation as claimed in one or more of claims 1 to 13, which comprises bringing

a) an ACE inhibitor or its physiologically tolerable salt and

b) a potassium channel modulator or its physiologically tolerable salt

into a suitable form for administration together with a physiologically acceptable carrier and, if appropriate, further auxiliaries and additives.

**15.** The use of a preparation as claimed in one or more of claims 1 to 13 in the treatment of high blood pressure, cardiac insufficiency and/or coronary heart disease.

**16.** A product containing

a) an ACE inhibitor of the formula I as in claim 1 or its physiologically tolerable salt and

b) a potassium channel modulator of the formula II as in claim 1 or its physiologically tolerable salt

as a combination preparation for simultaneous, separate or sequential administration in the treatment of high blood pressure.

**Claims for the following Contracting States : ES, GR**

**1.** A process for the preparation of a pharmaceutical preparation containing

a) an ACE inhibitor of the formula I

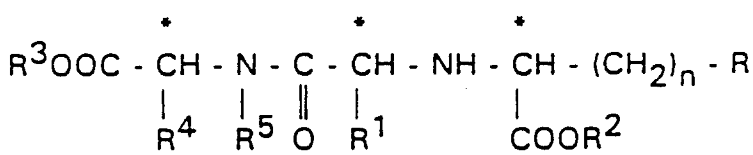

(I)

in which

| | |
|---|---|
| n | is 1 or 2, |
| R | denotes hydrogen,<br>an optionally substituted aliphatic radical having 1-8 carbon atoms,<br>an optionally substituted alicyclic radical having 3-9 carbon atoms,<br>an optionally substituted aromatic radical having 6-12 carbon atoms,<br>an optionally substituted araliphatic radical having 7-14 carbon atoms,<br>an optionally substituted alicyclic-aliphatic radical having 7-14 carbon atoms,<br>a radical $OR^a$ or $SR^a$, in which |
| $R^a$ | represents an optionally substituted aliphatic radical having 1-4 carbon atoms, an optionally substituted aromatic radical having 6-12 carbon atoms or an optionally substituted heteroaromatic radical having 5-12 ring atoms, |
| $R^1$ | denotes hydrogen, an optionally substituted aliphatic radical having 1-6 carbon atoms,<br>an optionally substituted alicyclic radical having 3-9 carbon atoms,<br>an optionally substituted alicyclic-aliphatic radical having 4-13 carbon atoms,<br>an optionally substituted aromatic radical having 6-12 carbon atoms,<br>an optionally substituted araliphatic radical having 7-16 carbon atoms,<br>an optionally substituted heteroaromatic radical having 5-12 ring atoms or<br>the side chain, protected if necessary, of a naturally occurring $\alpha$-amino acid, |
| $R^2$ and $R^3$ | are identical or different and denote hydrogen, an optionally substituted aliphatic radical having 1-6 carbon atoms,<br>an optionally substituted alicyclic radical having 3-9 carbon atoms,<br>an optionally substituted aromatic radical having 6-12 carbon atoms,<br>an optionally substituted araliphatic radical having 7-16 carbon atoms and |
| $R^4$ and $R^5$ | together with the atoms carrying them form a ring system from the group |

comprising tetrahydroisoquinoline, decahydroisoquinoline, octahydroindole and octahydrocyclopenta[b]-pyrrole;
or its physiologically tolerable salt
and

b) a potassium channel modulator of the formula II

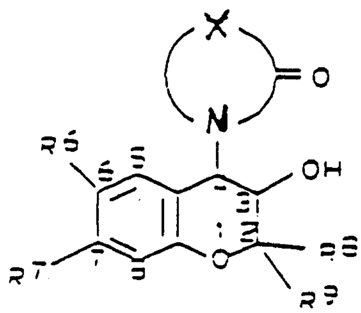

(II)

in which

| | |
|---|---|
| $R^6$ | represents CN, $NO_2$, $SO_n$-($C_1$-$C_6$)-alkyl or $SO_n$-Ar, where n is 1 or 2, Ar represents an aromatic or heteroaromatic system which is unsubstituted or substituted by 1 to 3 identical or different radicals from the series comprising ($C_1$-$C_2$)-alkyl, ($C_1$-$C_2$)-alkoxy, halogen, trifluoromethyl, CN, $NO_2$, CO-($C_1$-$C_2$)-alkyl or $SO_p$-($C_1$-$C_2$)-alkyl and p represents 1 or 2, |
| $R^7$ | represents hydrogen, hydroxyl, ($C_1$-$C_2$)-alkoxy, ($C_1$-$C_2$)-alkyl, halogen or $NR^{10}R^{11}$, where $R^{10}$ and $R^{11}$ are identical or different and represent hydrogen, ($C_1$-$C_2$)-alkyl or ($C_1$-$C_2$)-alkylcarbonyl, where the abovementioned meanings of $R^6$ and $R^7$ can also be interchanged, |
| $R^8$ and $R^9$ | are identical or different and represent alkyl having 1-4 carbon atoms, |
| X | represents a $(CH_2)_m$ chain which is unsubstituted or substituted by at least 1 and at most 2m-1 ($C_1$-$C_2$)-alkyl groups, and can be interrupted by a heteroatom Y with the meaning of O, $NR^{12}$ or S and $R^{12}$ denotes H or ($C_1$-$C_4$)-alkyl and m represents 2, 3 or 4, where the configuration of $C_3$ and $C_4$ is always opposite, or its physiologically tolerable salt, |

which comprises bringing
a) an ACE inhibitor or its physiologically tolerable salt and
b) a potassium channel modulator or its physiologically tolerable salt
into a suitable form for administration together with a physiologically acceptable carrier and, if appropriate, further auxiliaries and additives.

**2.** The process as claimed in claim 1, wherein in the ACE inhibitor of the formula I

| | |
|---|---|
| n | is 1 or 2 |
| R | denotes hydrogen,<br>alkyl having 1-8 carbon atoms,<br>alkenyl having 2-6 carbon atoms,<br>cycloalkyl having 3-9 carbon atoms,<br>aryl having 6-12 carbon atoms,<br>which can be monosubstituted, disubstituted or trisubstituted by ($C_1$-$C_4$)-alkyl, ($C_1$-$C_4$)-alkoxy, hydroxyl, halogen, nitro, amino, aminomethyl, ($C_1$-$C_4$)-alkylamino, di-($C_1$-$C_4$)-alkylamino, ($C_1$-$C_4$)-alkanoylamino, methylenedioxy, cyano and/or sulfamoyl,<br>alkoxy having 1-4 carbon atoms,<br>aryloxy having 6-12 carbon atoms,<br>which can be substituted as described above for aryl,<br>mono- or bicyclic heteroaryloxy having 5-7 or 8-10 ring atoms, of which 1 or 2 ring atoms are sulfur or oxygen atoms and/or 1 to 4 ring atoms are nitrogen,<br>which can be substituted as described above for aryl,<br>amino-($C_1$-$C_4$)-alkyl,<br>($C_1$-$C_4$)-alkanoylamino-($C_1$-$C_4$)-alkyl, |

$(C_7-C_{13})$-aroylamino-$(C_1-C_4)$-alkyl,
$(C_1-C_4)$-alkoxycarbonylamino-$(C_1-C_4)$-alkyl,
$(C_6-C_{12})$-aryl-$(C_1-C_4)$-alkoxycarbonylamino-$(C_1-C_4)$-alkyl,
$(C_6-C_{12})$-aryl-$(C_1-C_4)$-alkylamino-$(C_1-C_4)$-alkyl,
$(C_1-C_4)$-alkylamino-$(C_1-C_4)$-alkyl,
di-$(C_1-C_4)$-alkylamino-$(C_1-C_4)$-alkyl,
guanidino-$(C_1-C_4)$-alkyl,
imidazolyl, indolyl,
$(C_1-C_4)$-alkylthio,
$(C_1-C_4)$-alkylthio-$(C_1-C_4)$-alkyl,
$(C_6-C_{12})$-arylthio-$(C_1-C_4)$-alkyl,
which can be substituted in the aryl moiety as described above for aryl,
$(C_6-C_{12})$-aryl-$(C_1-C_4)$-alkylthio,
which can be substituted in the aryl moiety as described above for aryl,
carboxyl-$(C_1-C_4)$-alkyl,
carboxyl, carbamoyl,
carbamoyl-$(C_1-C_4)$-alkyl,
$(C_1-C_4)$-alkoxycarbonyl-$(C_1-C_4)$-alkyl,
$(C_6-C_{12})$-aryloxy-$(C_1-C_4)$-alkyl,
which can be substituted in the aryl moiety as described above for aryl or
$(C_6-C_{12})$-aryl-$(C_1-C_4)$-alkoxy,
which can be substituted in the aryl moiety as described above for aryl,

$R^1$ denotes hydrogen,
alkyl having 1-6 carbon atoms,
alkenyl having 2-6 carbon atoms,
alkynyl having 2-6 carbon atoms,
cycloalkyl having 3-9 carbon atoms,
cycloalkenyl having 5-9 carbon atoms,
$(C_3-C_9)$-cycloalkyl-$(C_1-C_4)$-alkyl,
$(C_5-C_9)$-cycloalkenyl-$(C_1-C_4)$-alkyl,
aryl, which is optionally partly hydrogenated, having 6-12 carbon atoms, which can be substituted as described above for R,
$(C_6-C_{12})$-aryl-$(C_1-C_4)$-alkyl or $(C_7-C_{13})$-aroyl-($C_1$ or $C_2$)-alkyl
which can both be substituted like the abovementioned aryl,
mono- or bicyclic heteroaryl, which is optionally partly hydrogenated, having 5-7 or 8-10 ring atoms, of which 1 or 2 ring atoms are sulfur or oxygen atoms and/or 1 to 4 ring atoms are nitrogen atoms,
which can be substituted like the abovementioned aryl or
the optionally protected side chain of a naturally occurring α-amino acid $R^1$-$CH(NH_2)$-COOH,

$R^2$ and $R^3$ are identical or different and denote hydrogen,
alkyl having 1-6 carbon atoms,
alkenyl having 2-6 carbon atoms,
di-$(C_1-C_4)$-alkylamino-$(C_1-C_4)$-alkyl,
$(C_1-C_5)$-alkanoyloxy-$(C_1-C_4)$-alkyl,
$(C_1-C_6)$-alkoxycarbonyloxy-$(C_1-C_4)$-alkyl,
$(C_7-C_{13})$-aroyloxy-$(C_1-C_4)$-alkyl,
$(C_6-C_{12})$-aryloxycarbonyloxy-$(C_1-C_4)$-alkyl,
aryl having 6-12 carbon atoms,
$(C_6-C_{12})$-aryl-$(C_1-C_4)$-alkyl,
$(C_3-C_9)$-cycloalkyl or
$(C_3-C_9)$-cycloalkyl-$(C_1-C_4)$-alkyl
and

$R^4$ and $R^5$ have the meaning indicated in claim 1.

3. The process as claimed in one or more of claims 1 to 3, wherein in the ACE inhibitor of the formula I
n is 1 or 2,
R denotes $(C_1-C_6)$-alkyl, $(C_2-C_6)$-alkenyl, $(C_3-C_9)$-cycloalkyl, amino-$(C_1-C_4)$-alkyl, ($C_2$-

$C_5$)-acylamino-($C_1$-$C_4$)-alkyl, ($C_7$-$C_{13}$)-aroylamino-($C_1$-$C_4$)-alkyl, ($C_1$-$C_4$)-alkoxycarbonylamino-($C_1$-$C_4$)-alkyl, ($C_6$-$C_{12}$)-aryl-($C_1$-$C_4$)-alkoxycarbonylamino-($C_1$-$C_4$)-alkyl, ($C_6$-$C_{12}$)-aryl which can be monosubstituted, disubstituted or trisubstituted by ($C_1$-$C_4$)-alkyl, ($C_1$-$C_4$)-alkoxy, hydroxyl, halogen, nitro, amino, ($C_1$-$C_4$)-alkylamino, di-($C_1$-$C_4$)-alkylamino and/or methylenedioxy, or 3-indolyl, in particular methyl, ethyl, cyclohexyl, tert.- butoxycarbonylamino-($C_1$-$C_4$)-alkyl, benzoyloxycarbonylamino-($C_1$-$C_4$)-alkyl or phenyl which can be monosubstituted or disubstituted by phenyl, ($C_1$-$C_2$)-alkyl, ($C_1$, or $C_2$)-alkoxy, hydroxyl, fluorine, chlorine, bromine, amino, ($C_1$-$C_4$)-alkylamino, di($C_1$-$C_4$)-alkylamino, nitro and/or methylenedioxy or, in the case of methoxy, trisubstituted,

$R^1$ denotes hydrogen or ($C_1$-$C_6$)-alkyl which can optionally be substituted by amino, ($C_1$-$C_6$)-acylamino or benzoylamino, ($C_2$-$C_6$)-alkenyl, ($C_3$-$C_9$)-cycloalkyl, ($C_5$-$C_9$)-cycloalkenyl, ($C_3$-$C_7$)-cycloalkyl-($C_1$-$C_4$)-alkyl, ($C_6$-$C_{12}$)-aryl or partly hydrogenated aryl, which in each case can be substituted by ($C_1$-$C_4$)-alkyl, ($C_1$ or $C_2$)-alkory or halogen, ($C_6$-$C_{12}$)-aryl-($C_1$-$C_4$)-alkyl or ($C_7$-$C_{13}$)-aroyl- ($C_1$-$C_2$)-alkyl, which can both be substituted in the aryl radical as defined in the foregoing, a mono- or bicyclic heterocyclic radical having 5 to 7 or 8 to 10 ring atoms, of which 1 or 2 ring atoms are sulfur or oxygen atoms and/or 1 to 4 ring atoms are nitrogen atoms, or a side chain of a naturally occurring, optionally protected α-amino acid, but in particular hydrogen, ($C_1$-$C_3$)-alkyl, ($C_2$ or $C_3$)-alkenyl, the optionally protected side chain of lysine, benzyl, 4-methoxybenzyl, 4-ethoxybenzyl, phenethyl, 4-aminobutyl or benzoylmethyl,

$R^2$ and $R^3$ denote identical or different radicals hydrogen, ($C_1$-$C_6$)-alkyl, ($C_2$-$C_6$)-alkenyl or ($C_6$-$C_{12}$)-aryl-($C_1$-$C_4$)-alkyl, but in particular hydrogen, ($C_1$-$C_4$)-alkyl or benzyl and

$R^4$ and $R^5$ have the meaning indicated in claim 1.

**4.** The process as claimed in one or more of claims 1 to 3, wherein the preparation contains 2-[N-(1-S-ethoxycarbonyl-3-phenylpropyl)-S-alanyl]-(1S,3S,5S)-2-azabicyclo[3.3.0]octane-3-carboxylic acid or a physiologically tolerable salt thereof.

**5.** The process as claimed in one or more of claims 1 to 3, wherein the preparation contains 1-[N-(1-S-ethoxycarbonyl-3-phenylpropyl)-S-alanyl]-(2S,3aR,7aS)-octahydro[1H]indole-2-carboxylic acid or a physiologically tolerable salt thereof.

**6.** The process as claimed in one or more of claims 1 to 3, wherein the preparation contains 2-[N-(1-S-ethoxycarbonyl-3-phenylpropyl)-S-alanyl]-1,2,3,4-tetrahydroisoquinoline-3-S-carboxylic acid or a physiologically tolerable salt thereof.

**7.** The process as claimed in one or more of claims 1 to 6, wherein the preparation contains a potassium channel modulator of the formula II in which
X represents a $(CH_2)_m$ chain which is unsubstituted or substituted by a ($C_1$-$C_2$)-alkyl group, and can be interrupted by a heteroatom Y which represents O, S or $NR^{12}$ with $R^{12}$ having the meaning of hydrogen or ($C_2$-$C_4$)-alkyl, and where m represents 2, 3 or 4,
or a physiologically tolerable salt thereof.

**8.** The process as claimed in one or more of claims 1 to 6, wherein the preparation contains a potassium channel modulator of the formula II in which X represents a $(CH_2)_m$ chain which is unsubstituted or substituted by a ($C_1$-$C_2$)-alkyl group where m represents 3 or 4,
or a physiologically tolerable salt thereof.

**9.** The process as claimed in one or more of claims 1 to 6, wherein the preparation contains a potassium channel modulator of the formula II in which
X represents a $(CH_2)_m$ chain, where m represents 3 or 4, which is substituted by a ($C_1$-$C_2$)-alkyl group on the carbon atom which is adjacent to the nitrogen atom of the lactam ring,
or a physiologically tolerable salt thereof.

**10.** The process as claimed in one or more of claims 1 to 6, wherein the preparation contains a potassium channel modulator of the formula II in which
X represents a $(CH_2)_m$ chain having m = 3 or 4 which is unsubstituted or substituted by a ($C_1$-$C_2$)-alkyl

group on the carbon atom which is adjacent to the nitrogen atom of the lactam ring, in particular in such a way that the configuration of this carbon atom is the same as that of the 4-carbon atom of the chroman system,
or a physiologically tolerable salt thereof.

**11.** The process as claimed in one or more of claims 1 to 10, wherein the preparation contains a potassium channel modulator of the formula II in which
$R^6$ represents $SO_2$-Ar with Ar having the meaning of phenyl which is unsubstituted or substituted by 1 to 3 substituents as mentioned in claim 8,
$R^7$ represents hydrogen or $OCH_3$,
$R^8$ and $R^9$ are identical or different and represent $(C_1$-$C_2)$-alkyl,
X represents a $(CH_2)_m$ chain having m = 3 or 4 which is unsubstituted or substituted by a $(C_1$-$C_2)$-alkyl group on the carbon atom which is adjacent to the nitrogen atom of the lactam ring, in particular in such a way that the configuration of this carbon atom is the same as that of the 4-carbon atom in the chroman system,
or a physiologically tolerable salt thereof.

**12.** The process as claimed in one or more of claims 1 to 11, wherein the preparation contains a potassium channel modulator of the formula II in which
$R^6$ represents CN or $SO_2$-$CH_3$ and $R^7$ represents hydrogen,
$R^8$ and $R^9$ are identical or different and represent alkyl having 1 or 2 carbon atoms,
X represents a $(CH_2)_m$ chain having m = 3 or 4 which is unsubstituted or substituted by a $(C_1$-$C_2)$-alkyl group on the carbon atom which is adjacent to the nitrogen atom of the lactam ring, in particular in such a way that the configuration of this carbon atom is opposite to that of the 4-carbon atom in the chroman system,
or a physiologically tolerable salt thereof.

**13.** The process as claimed in one or more of claims 1 to 12, wherein the preparation contains

| | |
|---|---|
| ramipril + | (±)-6-cyano-3,4-dihydro-2,2-dimethyl-trans-4-(2-oxo-1-pyrrolidinyl)-2H-benzo[b]-pyran-3-ol (chromakalim) or |
| ramipril + | 6-cyano-3-hydroxy-2,2-dimethyl-4-(5-methyl-2-oxo-1-pyrrolidinyl)-3,4-dihydro-2H-benzo[b]pyran or |
| ramipril + | 2,2-dimethyl-3-hydroxy-6-phenylsulfonyl-4-(2-oxo-1-pyrrolidinyl)-3,4-dihydro-2H-benzo[b]pyran or |
| trandolapril + | chromakalim or |
| trandolapril + | 6-cyano-3-hydroxy-2,2-dimethyl-4-(5-methyl-2-oxo-1-pyrrolidinyl)-3,4-dihydro-2H-benzo[b]pyran or |
| trandolapril + | 2,2-dimethyl-3-hydroxy-6-phenylsulfonyl-4-(2-oxo-1-pyrrolidinyl)-3,4-dihydro-2H-benzo[b]pyran or |
| quinapril + | chromakalim or |
| quinapril + | 6-cyano-3-hydroxy-2,2-dimethyl-4-(5-methyl-2-oxo-1-pyrrolidinyl)-3,4-dihydro-2H-benzo[b]pyran or |
| quinapril + | 2,2-dimethyl-3-hydroxy-6-phenysulfonyl-4-(2-oxo-1-pyrrolidinyl-3,4-dihydro-2H-benzo[b]pyran or |
| ramipril + | (3S,4R)-6-cyano-3,4-dihydro-2,2-dimethyl-4-(2-oxo-1-pyrrolidinyl)-2H-benzo[b]-pyran-3-ol or |
| trandolapril + | (3S,4R)-6-cyano-3,4-dihydro-2,2-dimethyl-4-(2-oxo-1-pyrrolidinyl)-2H-benzo[b]-pyran-3-ol or |
| quinapril + | (3S,4R)-6-cyano-3,4-dihydro-2,2-dimethyl-4-(2-oxo-1-pyrrolidinyl)-2H-benzo[b]-pyran-3-ol or |
| ramipril + | (3S,4R)-6-phenylsulfonyl-3,4-dihydro-2,2-dimethyl-4-(2-oxo-1-pyrrolidinyl)-2H-benzo[b]-pyran-3-ol or |
| trandolapril + | (3S,4R)-6-phenylsulfonyl-3,4-dihydro-2,2-dimethyl-4-(2-oxo-1- pyrrolidinyl)-2H-benzo[b]pyran-3-ol or |
| quinapril + | (3S,4R)-6-phenylsulfonyl-3,4-dihydro-2,2-dimethyl-4-(2-oxo-1-pyrrolidinyl)-2H-benzo[b]pyran-3-ol |

and in each case the physiologically tolerable salts of the individual components mentioned, if these can be formed.

**14.** The use of a preparation as claimed in one or more of claims 1 to 13 in the treatment of high blood pressure, cardiac insufficiency and/or coronary heart disease.

**15.** A process for the preparation of a product containing
a) an ACE inhibitor of the formula I as in claim 1 or its physiologically tolerable salt and
b) a potassium channel modulator of the formula II as in claim 1 or its physiologically tolerable salt
as a combination preparation for simultaneous, separate or sequential administration in the treatment of high blood pressure, which comprises bringing
a) an ACE inhibitor or its physiologically tolerable salt and
b) a potassium channel modulator or its physiologically tolerable salt
into a suitable form for administration together with a physiologically acceptable carrier and, if appropriate, further auxiliaries and additives.

**Revendications**

**Revendications pour les Etats contractants suivants : AT, BE, CH, DE, FR, GB, IT, LI, LU, NL, SE**

**1.** Composition pharmaceutique contenant
a) un inhibiteur de l'enzyme de conversion de l'angiotensine (ACE) de formule I

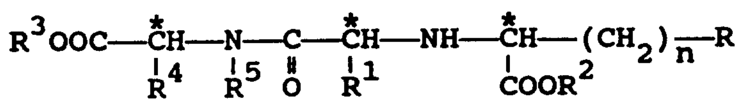

(I)

dans laquelle

| | |
|---|---|
| n | est 1 ou 2, |
| R | représente un atome d'hydrogène, un radical aliphatique éventuellement substitué ayant de 1 à 8 atomes de carbone, un radical alicyclique éventuellement substitué ayant de 3 à 9 atomes de carbone, un radical aromatique éventuellement substitué ayant de 6 à 12 atomes de carbone, un radical araliphatique éventuellement substitué ayant de 7 à 14 atomes de carbone, un radical alicycloaliphatique éventuellement substitué ayant de 7 à 14 atomes de carbone, un radical $OR^a$ ou $SR^a$, dans lequel $R^a$ représente un radical aliphatique éventuellement substitué ayant de 1 à 4 atomes de carbone, un radical aromatique éventuellement substitué ayant de 6 à 12 atomes de carbone ou un radical hétéroaromatique éventuellement substitué ayant de 5 à 12 atomes de carbone formant le cycle, |
| $R^1$ | représente un atome d'hydrogène, un radical aliphatique éventuellement substitué ayant de 1 à 6 atomes de carbone, un radical alicyclique éventuellement substitué ayant de 3 à 9 atomes de carbone, un radical alicycloaliphatique éventuellement substitué ayant de 4 à 13 atomes de carbone, un radical aromatique éventuellement substitué ayant de 6 à 12 atomes de carbone, un radical araliphatique éventuellement substitué ayant de 7 à 16 atomes de carbone, un radical hétéroaromatique éventuellement substitué ayant de 5 à 12 atomes de carbone formant le cyle, ou la chaîne latérale, protégée si nécessaire, d'un $\alpha$-aminoacide existant dans la nature, |
| $R^2$ et $R^3$ | sont identiques ou différents et représentent un atome d'hydrogène, un radical aliphatique éventuellement substitué ayant de 1 à 6 atomes de carbone, un radical alicyclique éventuellement substitué ayant de 3 à 9 atomes de carbone, un radical aromatique éventuellement substitué ayant de 6 à 12 atomes de carbone, un radical araliphatique éventuellement substitué ayant de 7 à 16 atomes de carbone, et |
| $R^4$ et $R^5$ | forment ensemble, avec les atomes qui les portent, un système cyclique choisi parmi les systèmes tétrahydroisoquinoléine, décahydroisoquinoléine, octahydroindo- |

le, octahydrocyclopenta[b]pyrrole,
ou un sel physiologiquement acceptable de celui-ci, et

b) un modulateur des canaux potassiques de formule II

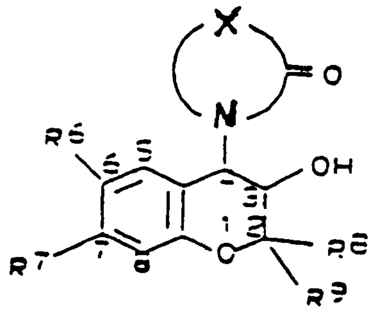

(II)

dans laquelle

- $R^6$ représente un groups CN, $NO_2$, $SO_n$-alkyle($C_1$-$C_6$), $SO_n$-Ar, n étant 1 ou 2, Ar représentant un système aromatique ou hétéroaromatique qui est non substitué ou substitué par 1 à 3 substituants, identiques ou différents, alkyle en $C_1$-$C_2$, alcoxy en $C_1$-$C_2$, halogène, trifluorométhyle, CN, $NO_2$, CO-alkyle($C_1$-$C_2$), $SO_p$-alkyle($C_1$-$C_2$), et p étant 1 ou 2,
- $R^7$ représente un atome d'hydrogène ou d'halogène ou un groupe hydroxy, alcoxy en $C_1$-$C_2$, alkyle en $C_1$-$C_2$ ou $NR^{10}R^{11}$, $R^{10}$ et $R^{11}$ étant identiques ou différents et représentant un atome d hydrogène ou un groupe alkyle en $C_1$-$C_2$ ou alkyl($C_1$-$C_2$)-carbonyle, les significations de $R^6$ et $R^7$ données plus haut pouvant être interverties,
- $R^8$ et $R^9$ sont identiques ou différents et représentent des groupes alkyle ayant de 1 à 4 atomes de carbone,
- X représente une chalne $(CH_2)_m$ qui est non substituée ou substituée par au moins 1 et au maximum 2m-1 groupes alkyle en $C_1$-$C_2$, et qui peut être interrompue par un hétéroatome Y représentant O, $NR^{12}$ ou S, et $R^{12}$ représentant H ou un groupe alkyle en $C_1$-$C_4$ et m représentant 2, 3 ou 4, les configurations en $C_3$ et $C_4$ étant toujours opposées,

ou un sel physiologiquement de celui-ci.

**2.** Composition selon la revendication 1, dans laquelle, dans l'inhibiteur d'ACE de formule I,

n = 1 ou 2,

R représente un atome d'hydrogène,
un radical alkyle ayant de 1 à 8 atomes de carbone,
alcényle ayant de 2 à 6 atomes de carbone,
cycloalkyle ayant de 3 à 9 atomes de carbone,
aryle ayant de 6 à 12 atomes de carbone,
qui peut être mono-, di- ou trisubstitué par un ou des atomes d'halogène ou groupes alkyle en $C_1$-$C_4$, alcoxy en $C_1$-$C_4$, hydroxy, nitro, amino, amino-méthyle, alkyl-($C_1$-$C_4$)-amino, dialkyl($C_1$-$C_4$)-amino, alcanoyl($C_1$-$C_4$)-amino, méthylènedioxy, cyano et/ou sulfamoyle,
un radical alcoxy ayant de 1 à 4 atomes de carbone,
aryloxy ayant de 6 à 12 atomes de carbone,
qui peut être substitué comme décrit plus haut pour aryle,
un radical hétéroaryloxy mono- ou bicyclique, ayant respectivement 5-7 ou 8-10 atomes formant le ou les cycles, dont 1 ou 2 atomes formant le cycle représentent des atomes de soufre ou d'oxygène et/ou 1 à 4 atomes formant le cycle représentent des atomes d'azote,
qui peut être substitué comme décrit plus haut pour aryle,
un radical amino-alkyle($C_1$-$C_4$),
alcanoyl($C_1$-$C_4$)amino-alkyle($C_1$-$C_4$),
aroyl($C_7$-$C_{13}$)amino-alkyle($C_1$-$C_4$),
alcoxy($C_1$-$C_4$)-carbonylamino-alkyle($C_1$-$C_4$),

aryl($C_6$-$C_{12}$)-alcoxy($C_1$-$C_4$)carbonylamino-alkyle($C_1$-$C_4$),
aryl($C_6$-$C_{12}$)-alkyl($C_1$-$C_4$)amino-alkyle($C_1$-$C_4$),
alkyl($C_1$-$C_4$)amino-alkyle($C_1$-$C_4$),
dialkyl($C_1$-$C_4$)amino-alkyle($C_1$-$C_4$),
guanidino-alkyle($C_1$-$C_4$),
imidazolyle, indolyle,
alkyl($C_1$-$C_4$)thio,
alkyl($C_1$-$C_4$)thio-alkyle($C_1$-$C_4$),
aryl($C_6$-$C_{12}$)thio-alkyle($C_1$-$C_4$),
qui peut être substitué sur le fragment aryle comme décrit plus haut pour aryle,
un radical aryl($C_6$-$C_{12}$)-alkyl($C_1$-$C_4$)thio,
qui peut être substitué sur le fragment aryle comme décrit plus haut pour aryle,
un radical carboxy-alkyle($C_1$-$C_4$),
carboxy, carbamoyle,
carbamoyl-alkyle($C_1$-$C_4$),
alcoxy($C_1$-$C_4$)-carbonyle-alkyle($C_1$-$C_4$),
aryloxy($C_6$-$C_{12}$)-alkyle($C_1$-$C_4$),
qui peut être substitué sur le fragment aryle comme décrit plus haut pour aryle, ou
un radical aryl($C_6$-$C_{12}$)-alcoxy($C_1$-$C_4$),
qui peut être substitué sur le fragment aryle comme décrit plus haut pour aryle,

$R^1$ représente un atome d'hydrogène,
un radical alkyle ayant de 1 à 6 atomes de carbone,
alcényle ayant de 2 à 6 atomes de carbone,
alcynyle ayant de 2 à 6 atomes de carbone,
cycloalkyle ayant de 3 à 9 atomes de carbone,
cycloalcényle ayant de 5 à 9 atomes de carbone,
cycloalkyl($C_3$-$C_9$)-alkyle($C_1$-$C_4$),
cycloalcényl($C_5$-$C_9$)-alkyle($C_1$-$C_4$),
un radical aryle ayant de 6 à 12 atomes de carbone, éventuellement partiellement hydrogéné, qui peut être substitué comme décrit plus haut à propos de R,
un radical aryl($C_6$-$C_{12}$)-alkyle($C_1$-$C_4$) ou aroyl($C_7$-$C_{13}$)-alkyle($C_1$ ou $C_2$)
qui peuvent l'un et l'autre être substitués comme le radical aryle précédent,
un radical hétéroaryle mono- ou bicyclique, éventuellement partiellement hydrogéné, ayant respectivement 5-7 ou 8-10 atomes formant le ou les cycles, dont 1 ou 2 atomes formant le cycle représentent des atomes de soufre ou d'oxygène et/ou 1 à 4 atomes formant le cycle représentent des atomes d'azote,
qui peut être substitué comme le radical aryle précédent, ou
la chaîne latérale éventuellement protégée d'un $\alpha$-aminoacide $R^1$-CH($NH_2$)-COOH existant dans la nature,

$R^2$ et $R^3$ sont identiques ou différents et représentent un atome d'hydrogène,
un radical alkyle ayant de 1 à 6 atomes de carbone,
alcényle ayant de 2 à 6 atomes de carbone,
dialkyl($C_1$-$C_4$)amino-alkyle($C_1$-$C_4$),
alcanoyloxy($C_1$-$C_5$)-alkyle($C_1$-$C_4$),
alcoxy($C_1$-$C_6$)-carbonyloxy-alkyle($C_1$-$C_4$),
aroyloxy($C_7$-$C_{13}$)-alkyle($C_1$-$C_4$),
aryloxy($C_6$-$C_{12}$)-carbonyloxy-alkyle($C_1$-$C_4$),
aryle ayant de 6 à 12 atomes de carbone,
aryl($C_6$-$C_{12}$)-alkyle($C_1$-$C_4$),
cycloalkyle en $C_3$-$C_9$ ou
cycloalkyl($C_3$-$C_9$)-alkyle($C_1$-$C_4$), et

$R^4$ et $R^5$ ont les significations données dans la revendication 1.

**3.** Composition selon une ou plusieurs des revendications 1 et 2, dans laquelle, dans l'inhibiteur d'ACE de formule I,

n = 1 ou 2,

R représente un radical alkyle en $C_1$-$C_6$, alcényle en $C_2$-$C_6$, cycloalkyle en $C_3$-$C_9$, amino-alkyle($C_1$-$C_4$), acyl($C_2$-$C_5$)-amino-alkyle($C_1$-$C_4$), aroyl($C_7$-$C_{13}$)amino-alkyle($C_1$-$C_4$),

alcoxy($C_1$-$C_4$)-carbonylamino-alkyle($C_1$-$C_4$), aryl($C_6$-$C_{12}$)-alcoxy($C_1$-$C_4$)carbonylamino-alkyle($C_1$-$C_4$), un radical aryle en $C_6$-$C_{12}$ qui peut être mono-, di- ou trisubstitué par un ou des atomes d'halogène ou groupes alkyle en $C_1$-$C_4$, alcoxy en $C_1$-$C_4$, hydroxy, nitro, amino, alkyl($C_1$-$C_4$)amino, dialkyl($C_1$-$C_4$)amino et/ou méthylènedioxy, ou le radical 3-indolyle, en particulier un radical méthyle, éthyle, cyclohexyle, tert-butoxycarbonylaminoalkyle($C_1$-$C_4$), benzyloxycarbonylamino-alkyle($C_1$-$C_4$) ou un radical phényle qui peut être mono- ou disubstitué par un ou des atomes de fluor, chlore ou brome ou groupes phényle, alkyle en $C_1$-$C_2$, alcoxy en $C_1$ ou $C_2$, hydroxy, amino, alkyl($C_1$-$C_4$)amino, dialkyl($C_1$-$C_4$)amino, nitro et/ou méthylènedioxy, ou trisubstitué dans le cas du groupe méthoxy,

$R^1$ représente un atome d'hydrogène ou un radical alkyle en $C_1$-$C_6$ qui peut éventuellement être substitué par un groupe amino, acyl($C_1$-$C_6$)amino ou benzoylamino, un radical alcényle en $C_2$-$C_6$, cycloalkyle en $C_3$-$C_9$, cycloalcényle en $C_5$-$C_9$, cycloalkyl($C_3$-$C_7$)-alkyle($C_1$-$C_4$), un radical aryle ou aryle partiellement hydrogéné en $C_6$-$C_{12}$, qui peut dans chaque cas être substitué par un atome d'halogène ou par un groupe alkyle en $C_1$-$C_4$ ou alcoxy en $C_1$ ou $C_2$, un radical aryl($C_6$-$C_{12}$)-alkyle($C_1$-$C_4$) ou aroyl($C_7$-$C_{13}$)-alkyle($C_1$-$C_2$) qui peuvent l'un et l'autre être substitués sur le fragment aryle comme défini précédemment, un radical hétérocyclique mono- ou bicyclique ayant respectivement 5-7 ou 8-10 atomes formant le ou les cycles, dont 1 ou 2 atomes formant le cyle représentent des atomes de soufre ou d'oxygène et/ou 1 à 4 atomes formant le cycle représentent des atomes d'azote ou une chaîne latérale d'un $\alpha$-aminoacide existant dans la nature, éventuellement protégé, mais en particulier un atome d'hydrogène ou un radical alkyle en $C_1$-$C_3$, alcényle en $C_2$ ou $C_3$, la chaîne latérale éventuellement protégée de la lysine, le radical benzyle, 4-méthoxybenzyle, 4-éthoxybenzyle, phénéthyle, 4-aminobutyle ou benzoylméthyle,

$R^2$ et $R^3$ sont identiques ou différents et représentent un atome d'hydrogène, un radical alkyle en $C_1$-$C_6$, alcényle en $C_2$-$C_6$ ou aryl($C_6$-$C_{12}$)-alkyle($C_1$-$C_4$), mais en particulier un atome d'hydrogène, un radical alkyle en $C_1$-$C_4$ ou benzyle, et

$R^4$ et $R^5$ ont les significations données dans la revendication 1.

**4.** Composition selon une ou plusieurs des revendications 1 à 3, contenant de l'acide 2-[N-(1-S-éthoxycarbonyl-3-phénylpropyl)-S-alanyl](1S,3S,5S)2-azabicyclo[3.3.0]-octane-3-carboxylique ou un sel physiologiquement acceptable de celui-ci.

**5.** Composition selon une ou plusieurs des revendications 1 à 3, contenant de l'acide 1-[N-(1-S-éthoxycarbonyl-3-phénylpropyl)-S-alanyl]-(2S,3aR,7aS)-octahydro[1H]indol-2-carboxylique ou un sel physiologiquement acceptable de celui-ci.

**6.** Composition selon une ou plusieurs des revendications 1 à 3, contenant de l'acide 2-[N-(1-S-éthoxycarbonyl-3-phénylpropyl)-S-alanyl]-1,2,3,4-tétrahydroisoquinoléine-3-S-carboxylique ou un de ses sels physiologiquement acceptable.

**7.** Composition selon une ou plusieurs des revendications 1 à 6, contenant un modulateur des canaux potassiques de formule II, dans lequel

X représente une chalne $(CH_2)_m$ qui est non substituée ou substituée par un groupe alkyle en $C_1$-$C_2$, et qui peut être interrompue par un hétéroatome Y qui représente O, S ou $NR^{12}$, $R^{12}$ représentant un atome d'hydrogène ou un groupe alkyle en $C_2$-$C_4$, et m étant 2, 3 ou 4,

ou un sel physiologiquement acceptable de celui-ci.

**8.** Composition selon une ou plusieurs des revendications 1 à 6, contenant un modulateur des canaux potassiques de formule II, dans lequel

X représente une chaîne $(CH_2)_m$ qui est non substituée ou substituée par un groupe alkyle en $C_1$-$C_2$, m étant 3 ou 4,

ou un sel physiologiquement acceptable de celui-ci.

**9.** Composition selon une ou plusieurs des revendications 1 à 6, contenant un modulateur des canaux potassiques de formule II, dans lequel

X représente un chaîne $(CH_2)_m$, m représentant 3 ou 4, qui est substituée par un groupe alkyle

en $C_1$-$C_2$ sur l'atome de carbone qui est contigu à l'atome d'azote du cycle lactame, ou un sel physiologiquement acceptable de celui-ci.

**10.** Composition selon une ou plusieurs des revendications 1 à 6, contenant un modulateur des canaux potassiques de formule II, dans lequel

X représente une chalne $(CH_2)_m$ dans laquelle m = 3 ou 4, qui est non substituée ou substituée par un groupe alkyle en $C_1$-$C_2$ sur l'atome de carbone qui est contigu à l'atome d'azote du cycle lactame, à savoir de sorte que la configuration de cet atome de carbone est la même que celle de l'atome de carbone $C_4$ du système chromane,

ou un sel physiologiquement acceptable de celui-ci.

**11.** Composition selon une ou plusieurs des revendications 1 à 6, contenant un modulateur des canaux potassiques de formule II, dans lequel

$R^6$ représente un radical $SO_2$-Ar, Ar représentant un groupe phényle qui est non substitué ou substitué par 1 à 3 substituants tels qu'indiqués dans la revendication 8,

$R^7$ représente un atome d'hydrogène ou le groupe $OCH_3$,

$R^8$ et $R^9$ sont identiques ou différents et représentent des radicaux alkyle en $C_1$-$C_2$,

X représente une chaîne $(CH_2)_m$ dans laquelle m = 3 ou 4, qui est non substituée ou substituée par un groupe alkyle en $C_1$-$C_2$ sur l'atome de carbone qui est contigu à l'atome d'azote du cycle lactame, à savoir de sorte que la configuration de cet atome de carbone est la même que celle de l'atome de carbone $C_4$ dans le système chromane, ou un sel physiologiquement acceptable de celui-ci.

**12.** Composition selon une ou plusieurs des revendications 1 à 6, contenant un modulateur des canaux potassiques de formule II, dans lequel

$R^6$ représente CN ou $SO_2$-$CH_3$ et $R^7$ représente un atome d'hydrogène,

$R^8$ et $R^9$ sont identiques ou différents et représentent des groupes alkyle ayant 1 ou 2 atomes de carbone,

X représente une chalne $(CH_2)_m$ dans laquelle m = 3 ou 4, qui est non substituée ou substituée par un groupe alkyle en $C_1$-$C_2$ sur l'atome de carbone qui est contiqu à l'atome d'azote du cycle lactame, à savoir de sorte que la configuration de cet atome de carbone est opposé à celle de l'atome de carbone $C_4$ dans le système chromane,

ou un sel physiologiquement acceptable de celui-ci.

**13.** Composition selon une ou plusieurs des revendications 1 à 6, contenant

du ramipril + du (±)-6-cyano-3,4-dihydro-2,2-diméthyl-trans-4-(2-oxo-1-pyrrolidinyl)-2H-benzo[b]pyranne-3-ol (chromakalim) ou

du ramipril + du 6-cyano-3-hydroxy-2,2-diméthyl-4-(5-méthyl-2-oxo-1-pyrrolidinyl)-3,4-dihydro-2H-benzo[b]-pyranne ou

du ramipril + du 2,2-diméthyl-3-hydroxy-6-phénylsulfonyl-4-(2-oxo-1-pyrrolidinyl)-3,4-dihydro-2H-benzo-[b]pyranne ou

du trandolapril + du chromakalim ou

du trandolapril + du 6-cyano-3-hydroxy-2,2-dimémthyl-4-(5-méthyl-2-oxo-1-pyrrolidinyl)-3,4-dihydro-2H-benzo[b]pyranne ou

du trandolapril + du 2,2-diméthyl-3-hydroxy-6-phényl-sulfonyl-4-(2-oxo-1-pyrrolidinyl)-3,4-dihydro-2H-benzo[b]pyranne ou

du quinapril + du chromokalim ou

du quinapril + du 6-cyano-3-hydroxy-2,2-diméthyl-4-(5-méthyl-2-oxo-1-pyrrolidinyl)-3,4-dihydro-2H-benzo[b]pyranne ou

du quinapril + du 2,2-diméthyl-3-hydroxy-6-phénylsulfonyl-4-(2-oxo-1-pyrrolidinyl)-3,4-dihydro-2H-benzo-[b]pyranne ou

du ramipril + du (3S,4R)-6-cyano-3,4-dihydro-2,2-diméthyl-4-(2-oxo-1-pyrrolidinyl)-2H-benzo-[b]pyranne-3-ol ou

du trandolapril + du (3S,4R)-6-cyano-3,4-dihydro-2,2-diméthyl-4-(2-oxo-1-pyrrolidinyl)-2H-benzo-[b]pyranne-3-ol ou

du quinapril + du (3S,4R)-6-cyano-3,4-dihydro-2,2-diméthyl-4-(2-oxo-1-pyrrolidinyl)-2H-benzo-[b]pyranne-3-ol ou

du ramipril + du (3S,4R)-6-phénylsulfonyl-3,4-dihydro-2,2-diméthyl-4-(2-oxo-1-pyrrolidinyl)-

| | |
|---|---|
| | 2H-benzo[b]-pyranne-3-ol ou |
| du trandolapril + | du (3S,4R)-6-phénylsulfonyl-3,4-dihydro-2,2-diméthyl-4-(2-oxo-1-pyrrolidinyl)-2H-benzo[b]pyranne-3-ol ou |
| du quinapril + | (3S,4R)-6-phénylsulfonyl-3,4-dihydro-2,2-diméthyl-4-(2-oxo-1-pyrrolidinyl)-2H-benzo-[b]pyranne-3-ol, |

ainsi que dans chaque cas les sels physiologiquement acceptables des composants individuels cités, lorsque ceux-ci peuvent être formés.

**14.** Procédé pour la préparation d'une composition selon une plusieurs des revendications 1 à 13, caractérisé en ce que l'on met sous une forme d'administration appropriée conjointement
a) un inhibiteur d'ACE ou un sel physiologiquement acceptable de celui-ci et
b) un modulateur des canaux potassiques ou un sel physiologiquement acceptable de celui-ci,
avec un véhicule physiologiquement acceptable et éventuellement d'autres adjuvants et additifs.

**15.** Utilisation d'une composition selon une ou plusieurs de revendications 1 à 13, dans le traitement de l'hypertension artérielle, de l'insuffisance cardiaque et/ou de la cardiopathie coronarienne.

**16.** Produit contenant
a) un inhibiteur d'ACE de formule I selon la revendication 1 ou un sel physiologiquement acceptable de celui-ci, et
b) un modulateur des canaux potassiques de formule II selon la revendication 1 ou un sel physiologiquement acceptable de celui-ci,
sous forme d'une association médicamenteuse pour administration simultanée, séparée ou échelonnée dans le temps, dans le traitement de l'hypertension artérielle.

**Revendications pour les Etats contractants suivants : ES, GR**

**1.** Procédé pour la préparation d'une composition pharmaceutique contenant
a) un inhibiteur de l'enzyme de conversion de l'angiotensine (ACE) de formule I

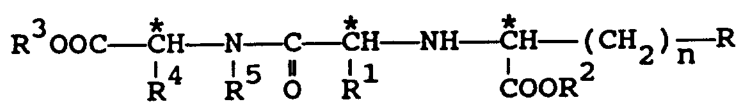

(I)

dans laquelle

n — est 1 ou 2,

R — représente un atome d'hydrogène,
un radical aliphatique éventuellement substitué ayant de 1 à 8 atomes de carbone,
un radical alicyclique éventuellement substitué ayant de 3 à 9 atomes de carbone,
un radical aromatique éventuellement substitué ayant de 6 à 12 atomes de carbone,
un radical araliphatique éventuellement substitué ayant de 7 à 14 atomes de carbone,
un radical alicycloaliphatique éventuellement substitué ayant de 7 à 14 atomes de carbone,
un radical $OR^a$ ou $SR^a$, dans lequel
$R^a$ représente un radical aliphatique éventuellement substitué ayant de 1 à 4 atomes de carbone, un radical aromatique éventuellement substitué ayant de 6 à 12 atomes de carhone ou un radical hétéroaromatique éventuellement substitué ayant de 5 à 12 atomes de carbone formant le cycle,

$R^1$ — représente un atome d'hydrogène,
un radical aliphatique éventuellement substitué ayant de 1 à 6 atomes de carbone,
un radical alicyclique éventuellement substitué ayant de 3 à 9 atomes de carbone,
un radical alicycloaliphatique éventuellement substitué ayant de 4 à 13 atomes de carbone,
un radical aromatique éventuellement substitué ayant de 6 à 12 atomes de carbone,
un radical araliphatique éventuellement substitué ayant de 7 à 16 atomes de carbone,

un radical hétéroaromatique éventuellement substitué ayant de 5 à 12 atomes de carbone formant le cyle, ou la chaîne latérale, protégée si nécessaire, d'un $\alpha$-aminoacide existant dans la nature,

$R^2$ et $R^3$ sont identiques ou différents et représentent un atome d'hydrogène, un radical aliphatique éventuellement substitué ayant de 1 à 6 atomes de carbone,
un radical alicyclique éventuellement substitué ayant de 3 à 9 atomes de carbone,
un radical aromatique éventuellement substitué ayant de 6 à 12 atomes de carbone,
un radical araliphatique éventuellement substitué ayant de 7 à 16 atomes de carbone, et

$R^4$ et $R^5$ forment ensemble, avec les atomes qui les portent, un système cyclique choisi parmi les systèmes tétrahydroisoquinoléine, décahydroisoquinoléine, octahydroindole, octahydrocyclopenta[b]pyrrole,
ou un sel physiologiquement de celui-ci, et

b) un modulateur des canaux potassiques de formule II

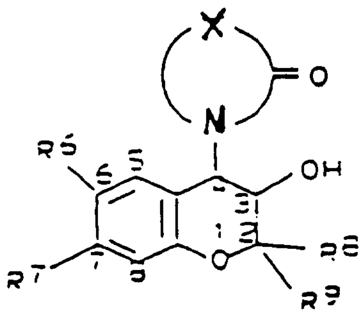

(II)

dans laquelle

$R^6$ représente un groupe CN, $NO_2$, $SO_n$-alkyle($C_1$-$C_6$), $SO_n$-Ar, n étant 1 ou 2, Ar représentant un système aromatique ou hétéroaromatique qui est non substitué ou substitué par 1 à 3 substituants, identiques ou différents, alkyle en $C_1$-$C_2$, alcoxy en $C_1$-$C_2$, halogène, trifluorométhyle, CN, $NO_2$, CO-alkyle($C_1$-$C_2$), $SO_p$-alkyle($C_1$-$C_2$), et p étant 1 ou 2,

$R^7$ représente un atome d'hydrogène ou d'halogène ou un groupe hydroxy, alcoxy en $C_1$-$C_2$, alkyle en $C_1$-$C_2$ ou $NR^{10}R^{11}$, $R^{10}$ et $R^{11}$ étant identiques ou différents et représentant un atome d'hydrogène ou un groupe alkyle en $C_1$-$C_2$ ou alkyl($C_1$-$C_2$)-carbonyle, les significations de $R^6$ et $R^7$ données plus haut pouvant être interverties,

$R^8$ et $R^9$ sont identiques ou différents et représentent des groupes alkyle ayant de 1 à 4 atomes de carbone,

X représente une chaine $(CH_2)_m$ qui est non substituée ou substituée par au moins 1 et au maximum 2m-1 groupes alkyle en $C_1$-$C_2$, et qui peut être interrompue par un hétéroatome Y représentant O, $NR^{12}$ ou S, et $R^{12}$ représentant H ou un groupe alkyle en $C_1$-$C_4$ et m représentant 2, 3 ou 4, les configurations en $C_3$ et $C_4$ étant toujours opposées,

ou un sel physiologiquement acceptable de celui-ci, caractérisé en ce que l'on met sous une forme d'administration appropriée conjointement

a) un inhibiteur d'ACE ou un sel physiologiquement acceptable de celui-ci et

b) un modulateur des canaux potassiques ou un sel physiologiquement acceptable de celui-ci,

avec un véhicule physiologiquement acceptable et éventuellement d'autres adjuvants et additifs.

**2.** Procédé selon la revendication 1, caractérisé en ce que, dans l'inhibiteur d'ACE de formule I,

n = 1 ou 2,

R représente un atome d'hydrogène,
un radical alkyle ayant de 1 à 8 atomes de carbone,
alcényle ayant de 2 à 6 atomes de carbone,
cycloalkyle ayant de 3 à 9 atomes de carbone,
aryle ayant de 6 à 12 atomes de carbone,
qui peut être mono-, di- ou trisubstitué par un ou des atomes d'halogène ou

groupes alkyle en $C_1$-$C_4$, alcoxy en $C_1$-$C_4$, hydroxy, nitro, amino, aminométhyle, alkyl-($C_1$-$C_4$)-amino, dialkyl($C_1$-$C_4$)-amino, alcanoyl($C_1$-$C_4$)-amino, méthylènedioxy, cyano et/ou sulfamoyle,
un radical alcoxy ayant de 1 à 4 atomes de carbone, aryloxy ayant de 6 à 12 atomes de carbone,
qui peut être substitué comme décrit plus haut pour aryle,
un radical hétéroaryloxy mono- ou bicyclique, ayant respectivement 5-7 ou 8-10 atomes formant le ou les cycles, dont 1 ou 2 atomes formant le cycle représentent des atomes de soufre ou d'oxygène et/ou 1 à 4 atomes formant le cycle représentent des atomes d'azote,
qui peut être substitué comme décrit plus haut pour aryle,
un radical amino-alkyle($C_1$-$C_4$),
alcanoyl($C_1$-$C_4$)amino-alkyle($C_1$-$C_4$),
aroyl($C_7$-$C_{13}$)amino-alkyle($C_1$-$C_4$),
alcoxy($C_1$-$C_4$)-carbonylamino-alkyle($C_1$-$C_4$),
aryl($C_6$-$C_{12}$)-alcoxy($C_1$-$C_4$)carbonylamino-alkyle($C_1$-$C_4$),
aryl($C_6$-$C_{12}$)-alkyl($C_1$-$C_4$)amino-alkyle($C_1$-$C_4$),
alkyl($C_1$-$C_4$)amino-alkyle($C_1$-$C_4$),
dialkyl($C_1$-$C_4$)amino-alkyle($C_1$-$C_4$),
guanidino-alkyle($C_1$-$C_4$),
imidazolyle, indolyle,
alkyl($C_1$-$C_4$)thio,
alkyl($C_1$-$C_4$)thio-alkyle($C_1$-$C_4$),
aryl($C_6$-$C_{12}$)thio-alkyle($C_1$-$C_4$),
qui peut être substitué sur le fragment aryle comme décrit plus haut pour aryle,
un radical aryl($C_6$-$C_{12}$)-alkyl($C_1$-$C_4$)thio,
qui peut être substitué sur le fragment aryle comme décrit plus haut pour aryle,
un radical carboxy-alkyle($C_1$-$C_4$),
carboxy, carbamoyle,
carbamoyl-alkyle($C_1$-$C_4$),
alcoxy($C_1$-$C_4$)-carbonyle-alkyle($C_1$-$C_4$),
aryloxy($C_6$-$C_{12}$)-alkyle($C_1$-$C_4$),
qui peut être substitué sur le fragment aryle comme décrit plus haut pour aryle, ou
un radical aryl($C_6$-$C_{12}$)-alcoxy($C_1$-$C_4$),
qui peut être substitué sur le fragment aryle comme décrit plus haut pour aryle,

$R^1$ représente un atome d'hydrogène,
un radical alkyle ayant de 1 à 6 atomes de carbone,
alcényle ayant de 2 à 6 atomes de carbone,
alcynyle ayant de 2 à 6 atomes de carbone,
cycloalkyle ayant de 3 à 9 atomes de carbone,
cycloalcényle ayant de 5 à 9 atomes de carbone,
cycloalkyl($C_3$-$C_9$)-alkyle($C_1$-$C_4$),
cycloalcényl($C_5$-$C_9$)-alkyle($C_1$-$C_4$),
un radical aryle ayant de 6 à 12 atomes de carbone, éventuellement partiellement hydrogéné, qui peut être substitué comme décrit plus haut à propos de R,
un radical aryl($C_6$-$C_{12}$)-alkyle($C_1$-$C_4$) ou aroyl($C_7$-$C_{13}$)-alkyle($C_1$ ou $C_2$)
qui peuvent l'un et l'autre être substitués comme le radical aryle précédent,
un radical hétéroaryle mono- ou bicyclique, éventuellement partiellement hydrogéné, ayant respectivement 5-7 ou 8-10 atomes formant le ou les cycles, dont 1 ou 2 atomes formant le cycle représentent des atomes de soufre ou d'oxygène et/ou 1 à 4 atomes formant le cycle représentent des atomes d'azote,
qui peut être substitué comme le radical aryle précédent, ou
la chaîne latérale éventuellement protégée d'un $\alpha$-aminoacide $R^1$-$CH(NH_2)$-$COOH$ existant dans la nature,

$R^2$ et $R^3$ sont identiques ou différents et représentent un atome d'hydrogène,
un radical alkyle ayant de 1 à 6 atomes de carbone,
alcényle ayant de 2 à 6 atomes de carbone,
dialkyl($C_1$-$C_4$)amino-alkyle($C_1$-$C_4$),

alcanoyloxy($C_1$-$C_5$)-alkyle($C_1$-$C_4$),
alcoxy($C_1$-$C_6$)-carbonyloxy-alkyle($C_1$-$C_4$),
aroyloxy ($C_7$-$C_{13}$)-alkyle($C_1$-$C_4$),
aryloxy($C_6$-$C_{12}$)-carbonyloxy-alkyle($C_1$-$C_4$),
aryle ayant de 6 à 12 atomes de carbone,
aryl($C_6$-$C_{12}$)-alkyle($C_1$-$C_4$),
cycloalkyle en $C_3$-$C_9$ ou
cycloalkyl($C_3$-$C_9$)-alkyle($C_1$-$C_4$), et

$R^4$ et $R^5$ ont les significations données dans la revendication 1.

3. Procédé selon une ou plusieurs des revendications 1 et 2, caractérisé en ce que, dans l'inhibiteur d'ACE de formule I,

n = 1 ou 2,

R représente un radical alkyle en $C_1$-$C_6$, alcényle en $C_2$-$C_6$, cycloalkyle en $C_3$-$C_9$, amino-alkyle($C_1$-$C_4$), acyl($C_2$-$C_5$)-amino-alkyle($C_1$-$C_4$), aroyl($C_7$-$C_{13}$)amino-alkyle($C_1$-$C_4$), alcoxy($C_1$-$C_4$)-carbonylamino-alkyle($C_1$-$C_4$), aryl($C_6$-$C_{12}$)-alcoxy ($C_1$-$C_4$)carbonylamino-alkyle($C_1$-$C_4$), un radical aryle en $C_6$-$C_{12}$ qui peut être mono-, di- ou trisubstitué par un ou des atomes d'halogène ou groupes alkyle en $C_1$-$C_4$, alcoxy en $C_1$-$C_4$, hydroxy, nitro, amino alkyl-($C_1$-$C_4$)amino, dialkyl($C_1$-$C_4$)amino et/ou méthylènedioxy, ou le radical 3-indolyle, en particulier un radical méthyle, éthyle, cyclohexyle, tert-butoxycarbonylaminoalkyle($C_1$-$C_4$), benzyloxycarbonylamino-alkyle($C_1$-$C_4$) ou un radical phényle qui peut être mono- ou disubstitué par un ou des atomes de fluor, chlore ou brome ou groupes phényle, alkyle en $C_1$-$C_2$, alcoxy en $C_1$ ou $C_2$, hydroxy, amino, alkyl($C_1$-$C_4$)amino, dialkyl($C_1$-$C_4$)amino, nitro et/ou méthylènedioxy, ou trisubstitué dans le cas du groupe méthoxy,

$R^1$ représente un atome d'hydrogène ou un radical alkyle en $C_1$-$C_6$ qui peut éventuellement être substitué par un groupe amino, acyl($C_1$-$C_6$)amino ou benzoylamino, un radical alcényle en $C_2$-$C_6$, cycloalkyle en $C_3$-$C_9$, cycloalcényle en $C_5$-$C_9$, cycloalkyl($C_3$-$C_7$)-alkyle($C_1$-$C_4$), un radical aryle ou aryle partiellement hydrogéné en $C_6$-$C_{12}$,qui peut dans chaque cas être substitué par un atome d'halogène ou par un groupe alkyle en $C_1$-$C_4$ ou alcoxy en $C_1$ ou $C_2$, un radical aryl($C_6$-$C_{12}$)-alkyle($C_1$-$C_4$) ou aroyl-($C_7$-$C_{13}$)-alkyle($C_1$-$C_2$) qui peuvent l'un et l'autre être substitués sur le fragment aryle comme défini précédemment, un radical hétérocyclique mono- ou bicyclique ayant respectivement 5-7 ou 8-10 atomes formant le ou les cycles, dont 1 ou 2 atomes formant le cyle représentent des atomes de soufre ou d'oxygène et/ou 1 à 4 atomes formant le cycle représentent des atomes d'azote, ou une chaîne latérale d'un $\alpha$-aminoacide existant dans la nature, éventuellement protégé, mais en particulier un atome d'hydrogène ou un radical alkyle en $C_1$-$C_3$, alcényle en $C_2$ ou $C_3$, la chaîne latérale éventuellement protégée de la lysine, le radical benzyle, 4-méthoxybenzyle, 4-éthoxybenzyle, phénéthyle, 4-aminobutyle ou bensoylméthyle,

$R^2$ et $R^3$ sont identiques ou différents et représentent un atome d'hydrogène, un radical alkyle en $C_1$-$C_6$, alcényle en $C_2$-$C_6$ ou aryl($C_6$-$C_{12}$)-alkyle($C_1$-$C_4$), mais en particulier un atome d'hydrogène, un radical alkyle en $C_1$-$C_4$ ou benzyle, et

$R^4$ et $R^5$ ont les significations données dans la revendication 1.

4. Procédé selon une ou plusieurs des revendications 1 à 3, caractérisé en ce que la composition contient de l'acide 2-[N(1-S-éthoxycarbonyl-3-phénylpropyl)-S-alanyl]-(2S,3S,5S)-2-azabicyclo[3.3.0]octane-3-carboxylique ou un sel physiologiquement acceptable de celui-ci.

5. Procédé selon une ou plusieurs des revendications 1 à 3, caractérisé en ce que la composition contient de l'acide 1-(N(1-S-éthoxycarbonyl-3-phénylpropyl)-S-alanyl]-(1S,3aR,7aS)-octahydro[1H]indol-2-carboxylique ou un sel physiologiquement acceptable de celui-ci.

6. Procédé selon une ou plusieurs des revendications 1 à 3, caractérisé en que que la composition contient de 1'acide 2-[N(1-S-éthoxycarbonyl-3-phénylpropyl)-S-alanyl]-1,2,3,4-tétrahydroisoquinoléine-3-S-carboxylique ou un de ses sels physiologiquement acceptable.

7. Procédé selon une ou plusieurs des revendications 1 à 6, caractérisé en ce que la composition contient un modulateur des canaux potassiques de formule II, dans lequel
   X représente une chaîne $(CH_2)_m$ qui est non substituée ou substituée par un groupe alkyle en $C_1$-$C_2$, et qui peut être interrompue par un hétéroatome Y qui représente O, S ou $NR^{12}$, $R^{12}$ représentant un atome d'hydrogène ou un groupe alkyle en $C_2$-$C_4$, et m étant 2, 3 ou 4,
   ou un sel physiologiquement acceptable de celui-ci.

8. Procédé selon une ou plusieurs des revendications 1 à 6, caractérisé en ce que la composition contient un modulateur des canaux potassiques de formule II, dans lequel
   X représente une chaine $(CH_2)_m$ qui est non substituée ou substituée par un groupe alkyle en $C_1$-$C_2$, m étant 3 ou 4,
   ou un sel physiologiquement acceptable de celui-ci.

9. Procédé selon une ou plusieurs des revendications 1 à 6, caractérisé en ce que la composition contient un modulateur des canaux potassiques de formule II, dans lequel
   X représente un chaîne $(CH_2)_m$, m représentant 3 ou 4, qui est substituée par un groupe alkyle en $C_1$-$C_2$ sur l'atome de carbone qui est contigu à l'atome d'azote du cycle lactame,
   ou un sel physiologiquement acceptable de celui-ci.

10. Procédé selon une ou plusieurs des revendications 1 à 6, caractérisé en ce que la composition contient un modulateur des canaux potassiques de formule II, dans lequel
   X représente une chaine $(CH_2)_m$ dans laquelle m = 3 ou 4, qui est non substituée ou substituée par un groupe alkyle en $C_1$-$C_2$ sur l'atome de carbone qui est contigu à l'atome d'azote du cycle lactame, à savoir de sorte que la configuration de cet atome de carbone est la même que celle de l'atome de carbone $C_4$ du système chromane,
   ou un sel physiologiquement acceptable de celui-ci.

11. Procédé selon une ou plusieurs des revendications 1 à 6, caractérisé en ce que la composition contient un modulateur des canaux potassiques de formule II, dans lequel
   $R^6$ représente un radical $SO_2$-Ar, Ar représentant un groupe phényle qui est non substitué ou substitué par 1 à 3 substituants tels qu'indiqués dans la revendication 8,
   $R^7$ représente un atome d'hydrogène ou le groupe $OCH_3$,
   $R^8$ et $R^9$ sont identiques ou différents et représentent des radicaux alkyle en $C_1$-$C_2$,
   X représente une chaîne $(CH_2)_m$ dans laquelle m = 3 ou 4, qui est non substituée ou substituée par un groupe alkyle en $C_1$-$C_2$ sur l'atome de carbone qui est contigu à l'atome d'azote du cycle lactame, à savoir de sorte que la configuration de cet atome de carbone est la même que celle de l'atome de carbone $C_4$ dans le système chromane, ou un sel physiologiquement acceptable de celui-ci.

12. Procédé selon une ou plusieurs des revendications 1 à 6, caractérisé en ce que la composition contient un modulateur des canaux potassiques de formule II, dans lequel
   $R^6$ représente CN ou $SO_2$-CH3 et $R^7$ représente un atome d'hydrogène,
   $R^8$ et $R^9$ sont identiques ou différents et représentent des groupes alkyle ayant 1 ou 2 atomes de carbone,
   X représente une chaîne $(CH_2)_m$ dans laquelle m = 3 ou 4, qui est non substituée ou substituée par un groupe alkyle en $C_1$-$C_2$ sur l'atome de carbone qui est contigu à l'atome d'azote du cycle lactame, à savoir de sorte que la configuration de cet atome de carbone est opposé à celle de l'atome de carbone $C_4$ dans le système chromane,
   ou un sel physiologiquement acceptable de celui-ci.

13. Procédé selon une ou plusieurs des revendications 1 à 12, caractérisé en ce que la composition contient
   du ramipril + du (±)-6-cyano-3,4-dihydro-2,2-diméthyl-trans-4-(2-oxo-1-pyrrolidinyl)-2H-benzo[b]pyranne-3-ol (chromakalim) ou
   du ramipril + du 6-cyano-3-hydroxy-2,2-diméthyl-4-(5-méthyl-2-oxo-1-pyrrolidinyl)-3,4-dihydro-2H-benzo[b]-pyranne ou
   du ramipril + du 2,2-diméthyl-3-hydroxy-6-phénylsulfonyl-4-(2-oxo-1-pyrrolidinyl)-3,4-dihydro-2H-benzo-[b]pyranne ou

| | |
|---|---|
| du trandolapril + | du chromakalim ou |
| du trandolapril + | du 6-cyano-3-hydroxy-2,2-dimémthyl-4-(5-méthyl-2-oxo-1-pyrrolidinyl)-3,4-dihydro-2H-benzo[b]pyranne ou |
| du trandolapril + | du 2,2-diméthyl-3-hydroxy-6-phényl-sulfonyl-4-(2-oxo-1-pyrrolidinyl)-3,4-dihydro-2H-benzo[b]pyranne ou |
| du quinapril + | du chromokalim ou |
| du quinapril + | du 6-cyano-3-hydroxy-2,2-diméthyl-4-(5-méthyl-2-oxo-1-pyrrolidinyl)-3,4-dihydro-2H-benzo[b]pyranne ou |
| du quinapril + | du 2,2-diméthyl-3-hydroxy-6-phénylsulfonyl-4-(2-oxo-1-pyrrolidinyl)-3,4-dihydro-2H-benzo-[b]pyranne ou |
| du ramipril + | du (3S,4R)-6-cyano-3,4-dihydro-2,2-diméthyl-4-(2-oxo-1-pyrrolidinyl)-2H-benzo-[b]pyranne-3-ol ou |
| du trandolapril + | du (3S,4R)-6-cyano-3,4-dihydro-2,2-diméthyl-4-(2-oxo-1-pyrrolidinyl)-2H-benzo-[b]pyranne-3-ol ou |
| du quinapril + | du (3S,4R)-6-cyano-3,4-dihydro-2,2-diméthyl-4-(2-oxo-1-pyrrolidinyl)-2H-benzo-[b]pyranne-3-ol ou |
| du ramipril + | du (3S,4R)-6-phénylsulfonyl-3,4-dihydro-2,2-diméthyl-4-(2-oxo-1-pyrrolidinyl)-2H-benzo[b]-pyranne-3-ol ou |
| du trandolapril + | du (3S,4R)-6-phénylsulfonyl-3,4-dihydro-2,2-diméthyl-4-(2-oxo-1-pyrrolidinyl)-2H-benzo[b]pyranne-3-ol ou |
| du quinapril + | (3S,4R)-6-phénylsulfonyl-3,4-dihydro-2,2-diméthyl-4-(2-oxo-1-pyrrolidinyl)-2H-benzo-[b]pyranne-3-ol, |

ainsi que dans chaque cas les sels physiologiquement acceptables des composants individuels cités, lorsque ceux-ci peuvent être formés.

**14.** Utilisation d'une composition selon une ou plusieurs de revendications 1 à 13, dans le traitement de l'hypertension artérielle, de l'insuffisance cardiaque et/ou de la cardiopathie coronarienne.

**15.** Procédé pour la préparation d'un produit contenant

a) un inhibiteur d'ACE de formule I selon la revendication 1 ou un sel physiologiquement acceptable de celui-ci, et

b) un modulateur des canaux potassiques de formule II selon la revendication 1 ou un sel physiologiquement acceptable de celui-ci,

sous forme d'une association médicamenteuse pour administration simultanée, séparée ou échelonnée dans le temps, dans le traitement de l'hypertension artérielle, caractérisé en ce que l'on met sous une forme d'administration appropriée conjointement

a) un inhibiteur d'ACE ou un sel physiologiquement acceptable de celui-ci et

b) un modulateur des canaux potassiques ou un sel physiologiquement acceptable de celui-ci,

avec un véhicule physiologiquement acceptable et éventuellement d'autres adjuvants et additifs.